(19) Europäisches Patentamt
European Patent Office
Office européen des brevets

(11) **EP 3 644 888 B1**

(12) **EUROPÄISCHE PATENTSCHRIFT**

(45) Veröffentlichungstag und Bekanntmachung des Hinweises auf die Patenterteilung:
**19.03.2025   Patentblatt 2025/12**

(21) Anmeldenummer: **18720556.2**

(22) Anmeldetag: **19.04.2018**

(51) Internationale Patentklassifikation (IPC):
*A61B 3/10* (2006.01)      *A61B 90/00* (2016.01)
*G01B 9/02091* (2022.01)      *G01B 9/02055* (2022.01)
*A61B 3/00* (2006.01)      *A61F 9/008* (2006.01)
*G01B 9/02004* (2022.01)

(52) Gemeinsame Patentklassifikation (CPC):
**A61B 90/37; A61B 3/0025; A61B 3/102;**
**A61F 9/00825; G01B 9/02064; G01B 9/02091;**
A61B 90/20; A61B 90/25; A61B 2090/3735;
A61F 2009/0087; G01B 9/02004; G01B 2290/35

(86) Internationale Anmeldenummer:
**PCT/EP2018/060114**

(87) Internationale Veröffentlichungsnummer:
**WO 2019/001795 (03.01.2019 Gazette 2019/01)**

(54) **VERFAHREN UND ANORDNUNG ZUR KORREKTUR EINER ABBILDUNG**

METHOD AND ARRANGEMENT FOR CORRECTING AN IMAGE

PROCÉDÉ ET SYSTÈME DE CORRECTION D'IMAGE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität:  **27.06.2017   DE 102017210779**

(43) Veröffentlichungstag der Anmeldung:
**06.05.2020   Patentblatt 2020/19**

(73) Patentinhaber: **Carl Zeiss Meditec AG**
**07745 Jena (DE)**

(72) Erfinder:
• **POMRAENKE, Robert**
**07743 Jena (DE)**

• **BERGT, Michael**
**99425 Weimar (DE)**
• **HAMANN, Thomas**
**07743 Jena (DE)**
• **EBERSBACH, Ralf**
**04626 Schmölln (DE)**

(74) Vertreter: **Rößner, Ulrike**
**Carl Zeiss AG**
**Patentabteilung Jena**
**Carl-Zeiss-Promenade 10**
**07745 Jena (DE)**

(56) Entgegenhaltungen:
WO-A1-2010/098204      WO-A2-2016/058931
DE-A1- 102010 019 657      DE-A1- 102014 115 153
DE-B3- 102004 028 204

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft ein Verfahren zur direkten Korrektur einer Abbildung, die auf einer Untersuchung eines Targets mit einem Untersuchungsstrahl einer Vorrichtung für die optische Kohärenztomographie basiert, wobei sich die optische Weglänge des Untersuchungsstrahls zum und/oder vom Target während der Erzeugung der Abbildung diskret oder kontinuierlich verändert. Die Erfindung betrifft weiterhin ein ophthalmologisches Untersuchungssystem.

Sowohl ophthalmologische Untersuchungssysteme als auch Therapiesysteme werden zunehmend komplexer. Ziel ist es in der Augenheilkunde zudem, Untersuchungssysteme in Therapiesysteme zu integrieren, um ein Therapieverfahren, wie beispielsweise laserchirurgische Verfahren, unter gleichzeitiger Möglichkeit der Beobachtung und letztlich unter Kontrolle, ggf. sogar unter einer aktiven Regelung mit Hilfe von Daten, die durch den Einsatz der Untersuchungssysteme ermittelt wurden, durchführen zu können.

Gleichzeitig besteht aber auch der Wunsch nach kleineren Systemen, die weniger der wertvollen Fläche in einem Operationssaal benötigen. Solche Systeme sollen zudem beweglich sein, so dass sie nach ihrem Einsatz "weggeräumt" werden können. Dies ist von Vorteil, wenn eine komplexe Therapie, insbesondere eine komplexe Operation, die mehrere Schritte aufweist und für die unterschiedliche Vorrichtungen eines komplexen Untersuchungs- und Therapiesystems eingesetzt werden sollen, durchgeführt werden soll.

Ein Beispiel dafür ist die Kataraktchirurgie, für die verschiedene Schnitte mit Hilfe eines Kurzpuls-Therapielaserstrahls durchgeführt werden müssen, aber es andererseits sowohl Untersuchungs- als auch Therapieschritte gibt, für die die Therapielaservorrichtung nicht benötigt wird. Die Therapielaservorrichtung - so sie nicht beweglich wäre - würde in diesen Schritten den zur Verfügung stehenden Operationsraum, und damit die Arbeitsfreiheit eines Chirurgen, erheblich einschränken.

Ein kompaktes System, insbesondere aber nicht ausschließlich für den Einsatz in der Kataraktchirurgie, wird in der WO 2016/058931 A2 beschrieben: Dabei handelt es sich um ein bewegliches System mit einem Gehäuse sowie mit an dem Gehäuse angeordneten Gelenkarmen, über die sowohl Untersuchungsstrahlung als auch Therapielaserstrahlung von ihrer Quelle im Gehäuse über die Gelenkarme auf ein Auge eines Patienten übertragen werden. Die Gelenkarme erlauben eine hohe Arbeitsfreiheit: Sie können "weggedreht" und geparkt werden, wenn sie nicht benötigt werden. Zudem erlauben Sie eine sehr flexible Anpassung der Arbeitsposition an die Lage des Patienten und an die Wünsche des behandelnden Arztes.

Da ein Therapielaserstrahl oder aber ein Untersuchungsstrahl den jeweiligen Gelenkarm und damit das System am beweglichen Ende des Gelenkarms verlässt, hat eine Änderung der Position des Gelenkarms ggf. Einfluss auf die Strahlführung, da dies insbesondere die Lagebeziehung einzelner Komponenten zueinander ändern und/oder eine Torsion (z.B. beim Einsatz von Lichtleitfasern) bewirken kann. Dies kann zu Änderungen des zu durchlaufenden optischen Wegs und/oder der Polarisation der jeweiligen Strahlung führen.

Zum anderen erfordert es ein solches kompaktes und bewegliches System, mit kompakten Optiken zu arbeiten, da ansonsten aufgrund eines relativ großen benötigten Bildfeldes der Objektivlinse - gerade für die Zwecke einer Katarakt-Behandlung, aber auch im Bereich der Korrektur von refraktiven Fehlern - die entsprechenden Gelenkarme sehr groß ausgeführt werden müssten und zudem ein wesentlich größeres Gewicht am freien Ende eines Gelenkarms darstellen würden. Um also ein solches System und insbesondere seine Arme klein zu halten, wird vorzugsweise eine lateral bewegliche Objektivlinse eingesetzt, deren Einzelbildfeld zwar kleiner als das in der Regel für eine Laserbehandlung benötigte Bildfeld ist. Dieses Bildfeld der Objektivlinse kann jedoch durch eine entsprechende Bewegung der Objektivlinse lateral verschoben werden, wodurch das Gesamtbildfeld beträchtlich vergrößert wird.

Durch diese bewegliche Objektivlinse kann sowohl der Untersuchungsstrahl während der Untersuchung eines Augengewebes als auch der Therapielaserstrahl während des chirurgischen Eingriffs am Auge geführt werden. Ggf werden auch beide gleichzeitig über diese lateral bewegliche Objektivlinse auf ein Augengewebe geleitet. Eine solche bewegliche Objektivlinse verändert jedoch beständig die optische Weglänge, die ein entsprechender Therapielaserstrahl oder ein Untersuchungsstrahl von der Strahlungsquelle auf seinem Weg zum Auge (und für den Untersuchungsstrahl ggf. auch wieder von einem Augengewebe zurück bis zu einer Analysevorrichtung) zu durchlaufen hat. Auch kann sich auf diesem Weg - beispielsweise durch Drehung der Gelenkarme - die Polarisation eines Therapielaserstrahls oder eines Untersuchungsstrahls ändern.

Eine solche Änderung der zu durchlaufenden optischen Weglänge sowie der Polarisation ist für einen Therapielaserstrahl - beispielsweise eines Kurzpuls-Lasers, insbesondere eines Femtosekunden-Lasers -, mit dem ein organisches Gewebe - vorzugsweise ein Augengewebe - mittels Photodisruption, Ablation oder Koagulation bearbeitet werden soll, unerheblich. Für den Therapielaserstrahl ist einzig die Zuverlässigkeit der Positionierung des Fokus bzw. der Fokusverschiebung ausschlaggebend. Die Bewegung der Gelenkarme kann natürlich auch auf diese Positionierung einen Einfluss haben. Hierfür wird in der WO 2016/058931 A2 ein System zur Stabilisierung eines Strahldurchgangs durch den Gelenkarm vorgeschlagen.

Hat jedoch der durchlaufene optische Weg und/oder die Polarisation einer Strahlung Einfluss auf das Ergebnis einer Untersuchung oder Therapie, dann verfälschen deren Änderungen aufgrund einer Bewegung des Systems ggf. diese Ergebnisse. Dies ist der Fall in einer Untersuchung eines organischen Gewebes, insbesondere eines Augengewebes, mittels optischer Köhärenztomographie (OCT). Systeme, in denen sich der durchlaufene optische Weg und/oder die Polarisation der Strahlung durch einfache Bewegungen im System während der Untersuchungen zum Zwecke der Messung selbst ändert, werden in den Dokumenten WO 2016/058931 A2, DE 10 2014 115 153 A1, WO 2010/098204 A1 bzw. DE 10 2010 019657 A1 beschrieben.

Ein Untersuchungssystem, das nach den Prinzipien der optischen Köhärenztomographie arbeitet, das jedoch bewegliche Teilsysteme (wie beispielsweise bewegliche Gelenkarme und/oder eine bewegliche Objektivlinse) enthält, wie es insbesondere im Dokument WO 2016/058931 A2 beschrieben ist, erzeugt demnach in unterschiedlichen Stellungen seiner beweglichen Komponenten unterschiedliche Abbildungen ein und desselben Targets, die durch Änderungen des durchlaufenen optischen Wegs und/oder der Polarisation des Untersuchungsstrahls aufgrund der entsprechenden zusätzlichen Bewegungen (z.B. der Gelenkarme der WO 2016/058931 A2) zusätzlich modifiziert werden, also beispielsweise verzerrt bzw. anderweitig verfälscht werden.

In der DE 10 2004 028 204 B3 wird ein Verfahren zur Signalauswertung bei einer time-domain OCT vorgestellt, welches eine Änderungsgeschwindigkeit der zeitlich veränderlichen optischen Weglänge eines Phasenmodulators im Referenzarm in einem Messzyklus ermittelt und in folgenden Messzyklen zur Demodulation des OCT-Messsignals verwendet.

Aufgabe ist es deshalb, ein Verfahren und entsprechende Systeme aufzuzeigen, mit denen in einem Untersuchungssystem, das eine Vorrichtung für die optische Kohärenztomographie umfasst, und das eine Strahlführungsvorrichtung enthält, in der eine optische Weglänge und/oder eine Polarisation eines Untersuchungsstrahls veränderlich ist, eine unverfälschte, also durch die Änderung der optischen Weglänge und/oder der Polarisation nicht modifizierte Abbildung (einer Struktur) eines Targets erzeugt werden kann.

Diese Aufgabe wird gelöst durch ein Verfahren zur Korrektur einer Abbildung nach Anspruch 1 und ein ophthalmologische Untersuchungssystem nach Anspruch 5.

In einem Verfahren zur Korrektur einer Abbildung, die auf einer Untersuchung eines Targets mit einem Untersuchungsstrahl einer Vorrichtung für die optische Kohärenztomographie (OCT) basiert, wird eine Abbildung einer Struktur des Targets oder Bilddaten einer Abbildung einer Struktur des Targets verwendet, zu deren Erzeugung eine zurückgelegte optische Weglänge von einer Quelle des Untersuchungsstrahls zu der Struktur des Targets und/oder von der Struktur des Targets zu einer Analysevorrichtung und/oder eine Polarisation des Untersuchungsstrahls ausgewertet wird. Die Auswertung wiederum basiert auf einem Vergleich des Untersuchungsstrahls mit einem Referenzstrahl: Während der Untersuchungsstrahl den Weg von der Quelle zum Target und vom Target zur Analysevorrichtung zurücklegt, wird der Referenzstrahl - in der Regel mit einem Strahlteiler - aus dem Untersuchungsstrahl auf seinem Weg zum Target "abgeteilt" und stattdessen in einen Referenzarm geleitet. Der vom Target zur Analysevorrichtung zurückkommende Untersuchungsstrahl wird dann mit dem aus dem Referenzarm zur Analysevorrichtung zurückkommenden Referenzstrahl verglichen, indem Untersuchungsstrahl und Referenzstrahl - mittels eines Strahlvereinigers ("beam combiner") - vor der Analysevorrichtung zur Interferenz gebracht werden. Dieser "Vergleich" (durch Erzeugung eines Interferenzsignals) von Untersuchungsstrahl und Referenzstrahl kann je nach OCT-Methode sehr unterschiedlich verlaufen: Bei einem TD ("time domain") - OCT wird der Referenzarm in der Länge variiert und kontinuierlich die Intensität der Interferenz gemessen, wobei das Spektrum keine Rolle spielt. Beim FD ("frequency domain") - OCT hingegen wird die Interferenz einzelner spektraler Komponenten analysiert. Dabei bleibt die Länge des Referenzarms konstant, jedoch benötigt es ein Spektrometer zur Detektion. Beim SS ("swept source") - OCT hingegen wird die Wellenlänge der Strahlungsquelle zeitlich durchgestimmt. Damit ist weder eine Variation der Referenzarmlänge nötig, noch eine spektrale Detektion.

Ein Target kann dabei ein organisches Material sein, insbesondere ein Gewebe eines Auges, beispielsweise die Linse, der Linsensack, der Glaskörper, die Kornea bzw. die Sklera. Im Allgemeinen kann das Target aber jegliches Material sein, das von einem Untersuchungsstrahl einer Vorrichtung für die optische Kohärenztomographie durchdrungen werden kann, d.h. für diese Strahlung transparent ist. Ein zu untersuchendes Target weist dabei Strukturen - an seiner Oberfläche und/oder in seinem Volumen - auf, die bei der Untersuchung bestimmt werden sollen.

Handelt es sich um ein absolut statisches Untersuchungssystem, mit dem eine OCT-Untersuchung durchgeführt wird, dann ist für ein und dieselbe Position in einem Volumen eines Targets für den Untersuchungsstrahl stets derselbe optische Weg von der Strahlungsquelle zu dieser Position des Targets (und vom Target zu einer Analysevorrichtung) zurückzulegen. Bei einer Untersuchung der Struktur eines Targets mittels A-Scan bzw. B-Scan einer optischen Kohärenztomographie in diesem ansonsten unbeweglichen System wird diese Struktur folglich korrekt abgebildet und braucht nicht korrigiert, insbesondere nicht "entzerrt", verschoben oder kontrastverbessert zu werden.

Jedoch kommt es aus verschiedenen Gründen vor, dass sich die optische Weglänge des Untersuchungsstrahls von der Strahlungsquelle zum und/oder vom Target zur Analysevorrichtung und/oder seine Polarisation im Verlaufe der Erzeugung der Abbildung der Struktur des Targets diskret oder kontinuierlich ändert. Dabei ist mit der diskreten oder kontinuierlichen Änderung der optischen Weglänge, die der Untersuchungsstrahl zum und/oder vom Target zu durchlaufen hat, die optische Weglänge jeweils zu einer konkreten Position auf oder im Volumen eines Targets gemeint: Es ändert sich also die relative Lage zwischen dem Target als solchem und einer Vorrichtung für die optische Kohärenztomographie bzw. ihrer Komponenten. Eine solche Änderung der optischen Weglänge, die der Untersuchungsstrahl von der Strahlungsquelle zu einer und/oder von einer konkreten Position des Targets zu einer Analysevorrichtung zu durchlaufen hat, und/oder eine Änderung der Polarisation des Untersuchungsstrahls ist beispielsweise aufgrund eines beweglichen Untersuchungssystems, insbesondere eines Untersuchungssystems, das in seiner Strahlführungsvorrichtung bewegliche Komponenten enthält, gegeben.

Dabei ist für die Abbildung, die auf einer Untersuchung eines Targets mit einem Untersuchungsstrahl einer Vorrichtung für die optische Kohärenztomographie (OCT) basiert, die Änderung der optischen Weglänge und/oder der Polarisation vom Untersuchungsstrahl in dem Bereich wichtig, wo er tatsächlich getrennt vom Referenzstrahl verläuft - und betrifft für OCT-Verfahren also die Strahlführung des Untersuchungsstrahls ab einem strahlteilenden Element hinter der Strahlungsquelle (mittels dem aus einem gemeinsamen Strahl, der aus der Strahlungsquelle austritt, erst in Untersuchungsstrahl und Referenzstrahl geteilt wird) bis zum Target und vom Target bis zu einem strahlvereinenden Element (mittels dem Untersuchungsstrahl und Referenzstrahl zusammengeführt werden, um zu interferieren) vor einer Analysevorrichtung. Im Folgenden wird dieser Bereich, in dem der Untersuchungsstrahl tatsächlich vom Referenzstrahl getrennt existiert, bezeichnet als "optische Weglänge des Untersuchungsstrahls (zum und/oder vom Target)". Die Änderung der optischen Weglänge für ein OCT Verfahren entspricht also der Änderung der Differenz zwischen der Länge des optischen Weges des Untersuchungsstrahls und der Länge des optischen Weges des Referenzstrahls (jeweils zu einer konkreten Position auf oder im Volumen des Targets).

Wird nun beispielsweise ein A-Scan bzw. ein B-Scan einer optischen Kohärenztomographie in einem beweglichen Untersuchungssystem durchgeführt, so überlagert eine Änderung der optischen Weglänge die "normale" OCT-Scanbewegung. In der Regel erfolgt eine solche Änderung der optischen Weglänge nur im Strahlengang des Untersuchungsstrahls: Der Strahlengang des Referenzstrahls ist üblicherweise so angeordnet, dass er von solchen Änderungen ausgenommen ist bzw. mögliche Änderungen überwacht und korrigiert werden. Er kann in einem stabilen Bereich des Untersuchungssystems verbleiben, da er nicht auf das Target gerichtet ist. Gibt es jedoch Änderungen der optischen Weglänge im Strahlengang des Referenzstrahls, die auf andere Einflüsse zurückzuführen sind als die Funktion des (TD)-OCT, dann müssen auch diese Einflüsse berücksichtigt werden. Beispielsweise werden Weglängenänderungen z.B. aufgrund von Temperaturänderungen im Bereich des Strahlengangs des Referenzstrahls, entsprechend detektiert und korrigiert.

Der Einfluss einer Änderung der optischen Weglänge könnte natürlich auch "umgangen" werden, wenn die Trennung in Untersuchungsstrahl und Referenzstrahl mittels eines Strahlteilers bzw. deren Wiedervereinigung im Strahlengang erst nach den beweglichen Bereichen (von der Quelle der Untersuchungsstrahlung in Richtung Target gesehen), die eine Änderung der optischen Weglänge bewirken, erfolgte. Hierfür ist jedoch in aller Regel kein Platz, da sich die beweglichen Bereiche des ophthalmologischen Untersuchungssystems bis kurz vor das Auge erstrecken.

Durch die Änderung der optischen Weglänge im Strahlengang des Untersuchungsstrahls und/oder seiner Polarisation wird die entsprechende Abbildung verändert bzw. modifiziert: Dabei führt die Änderung der optischen Weglänge zu einer Verschiebung und Verzerrung der Abbildung, die Änderung der Polarisation hingegen zu einer Kontraständerung bzw. Kontrastverschlechterung im Wesentlichen aufgrund einer Verschlechterung des Signal-Rausch-Verhältnisses. Der Begriff der Modifizierung der Abbildung, wie er hier verwendet wird, beinhaltet also eine Änderung in der Lage der Strukturen in der Abbildung im Vergleich zu deren wahrer Lage wie auch eine Kontraständerung, wenn beide Faktoren - die Änderung der optischen Weglänge und der Polarisation - eine Rolle spielen.

Eine Änderung der optischen Weglänge im Verlaufe der Erzeugung der Abbildung bzw. der Bilddaten der Abbildung kann dabei kontinuierlich erfolgen - beispielsweise aufgrund einer kontinuierlichen Bewegung des Untersuchungssystems. Oder aber sie erfolgt diskret, beispielsweise durch eine diskrete Änderung der Einstellungen zwischen zwei Abbildungsintervallen. Gleiches gilt auch für die Änderung der Polarisation.

Erfindungsgemäß ist nun die Änderung der optischen Weglänge des Untersuchungsstrahls zum und/oder vom Target und/oder die Änderung seiner Polarisation bekannt oder wird gemessen:

Beim Auftreten solch einer (kontinuierlichen oder diskreten) Änderung der optischen Weglänge des Untersuchungsstrahls zum und/oder vom Target und/oder einer Änderung seiner Polarisation während einer entsprechenden Untersuchung hängt es nun von der Art und Weise der Bewegung im Untersuchungssystem ab, ob die jeweilige Änderung der optischen Weglänge und/oder der Polarisation des Untersuchungsstrahls berechenbar oder vorbestimmbar ist und somit als "bekannt" bezeichnet werden kann: Beispielsweise können in einem beweglichen Untersuchungssystem die Änderungen der optischen Weglänge und/oder der Polarisation für alle dem Untersuchungssystem möglichen Bewegungspositionen vorbestimmt und in einer Tabelle ("look-up table") hinterlegt werden. Dann braucht während der eigentlichen OCT-Untersuchung der Struktur eines Targets nur die jeweilige Bewegungsposition bzw. die jeweilige Positionsänderung (also beispielsweise eine Verschiebung oder Drehung der Arme) im Vergleich zur vorherigen Bewegungsposition des Untersuchungssystems registriert zu werden, um mittels der Daten aus der Tabelle die jeweilige Änderung der optischen Weglänge und/oder der Polarisation zu bestimmen.

Oder aber es ist aufgrund einer physikalischen Beschreibbarkeit der Bewegung im Untersuchungssystem möglich, insbesondere die Änderung der optischen Weglänge in Abhängigkeit der jeweiligen Bewegungsposition zu berechnen, so dass auch hier während der eigentlichen OCT-Untersuchung der Struktur eines Targets nur die jeweilige Bewegungsposition bzw. eine Verschiebung im Vergleich zur vorherigen Bewegungsposition registriert werden muss, um die Änderung der optischen Weglänge zu ermitteln.

Ist weder eine Vorbestimmung noch eine Berechnung möglich oder angeraten, so wird die Änderung der optischen Weglänge mittels einer zusätzlichen Referenzmessung bestimmt. Hierzu kann zum Beispiel eine bekannte Fläche oder Struktur aus dem Gesamtsystem als Referenzfläche bzw. Referenzstruktur genutzt werden. Diese sollte vorzugsweise im Strahlengang nahe der Lage des Auges in einer Position angeordnet sein, in der eine einfache Abbildung möglich ist, auf jeden Fall aber hinter dem Bereich, dessen Bewegung die optische Weglänge verändert. Wenn die korrekte Lage der Referenzfläche bzw. der Referenzstruktur bekannt ist, kann das OCT-Bild so verschoben werden bzw. die Bilddaten der Abbildung so korrigiert werden, dass die Referenzfläche bzw. die Referenzstruktur an der erwarteten Stelle liegt.

Die Änderung der Polarisation des Untersuchungsstrahls kann durch Messung im Untersuchungsstrahl (d.h. im "Signalarm") mit Hilfe eines zusätzlichen Strahlteilers erfolgen. Man erzeugt dafür also im Untersuchungsstrahl mit Hilfe eines Strahlteilers nach den Elementen, die die Polarisation des Untersuchungsstrahls ändern, einen neuen "Messstrahl". Die Änderung der Polarisation allein kann beispielsweise in einfacher Weise mittels einer Referenzanordnung gemessen werden, die einen Strahlteiler im Strahlengang des Untersuchungsstrahls sowie eine Photodiode hinter einem Polarisator enthält: Der Strahlteiler lenkt dabei einen Teil der Strahlung auf die Photodiode.

Weiterhin erfindungsgemäß wird nun die bekannte oder gemessene Änderung der optischen Weglänge und/oder der Polarisation des Untersuchungsstrahls zur Einstellung eines verstellbaren Korrekturelements oder aber zur direkten Korrektur der Abbildung bzw. der Bilddaten der Abbildung verwendet, derart, dass der Einfluss der Änderung der optische Weglänge des Untersuchungsstrahls zum und/oder vom Target und/oder seiner Polarisation auf die Abbildung ausgeglichen wird.

Mittels eines verstellbaren Korrekturelements wird darauf abgezielt, die erfolgte Änderung der optischen Weglänge, die der Untersuchungsstrahl zu durchlaufen hat, aufzuheben, d.h., in Größenordnung der Änderung der optischen Weglänge, jedoch in entgegengesetzter Richtung, der Änderung der optischen Weglänge entgegenzuwirken.

Ein solches verstellbares Korrekturelement ist dabei im Strahlengang des Untersuchungsstrahls oder alternativ in den Strahlengang des Referenzstrahls einsetzbar: Beim Einsatz im Strahlengang des Referenzstrahls muss jedoch ein Korrekturelement so wirken, dass es die gleiche Änderung der optischen Weglänge bzw. der Polarisation bewirkt, die auch im Untersuchungsstrahl wirksam ist.

Die bekannte oder gemessene Änderung der optischen Weglänge und/oder Polarisation des Untersuchungsstrahls wird zu einer direkten Korrektur der Abbildung bzw. zu einer direkten Korrektur der Bilddaten der Abbildung eingesetzt. Dabei wird die bekannte oder gemessene Änderung der optischen Weglänge des Untersuchungsstrahls auf rechnerische Art und Weise berücksichtigt, um die Modifizierung der Abbildung durch die Änderung der optischen Weglänge des Untersuchungsstrahls, insbesondere deren Verschiebung und Verzerrung, zu korrigieren und damit aufzuheben. Für die Polarisation ist jedoch eine Korrektur durch ein verstellbares Korrekturelement bei Weitem einer rechnerischen Nachbearbeitung bevorzugt.

Bei dem erfindungsgemäßen Verfahren handelt es sich also um ein Verfahren zur direkten Korrektur einer Abbildung, das auf ein Verfahren zur Erzeugung einer Abbildung bzw. der Bilddaten der Abbildung, die unter nichtstatischen Bedingungen erzielt werden, angewendet wird.

Ein dem erfindungsgemäßen Verfahren äquivalentes Verfahren kann des Weiteren auf jegliche Untersuchung eines Targets mit einem Untersuchungsstrahl nach einer Untersuchungsmethode angewendet werden, bei der der durchlaufene optische Weg und/oder die Polarisation des Strahls Einfluss auf das Ergebnis der Untersuchung hat.

In einer besonderen Ausführungsform des Verfahrens zur direkten Korrektur einer Abbildung wird der Untersuchungsstrahl mittels Lichtleitfaser und/oder frei durch ein in seiner internen Lagebeziehung seiner Komponenten variierbares System geleitet und eine während einer Lagebeziehungsänderung des Systems entstehende Änderung der Polarisation des Untersuchungsstrahls durch zwei bewegliche Paddles oder durch eine Phasenverzögerungsplatte ("Wave Plate") korrigiert, wobei die Paddels für eine Korrektur der Polarisation in einer Lichtleitfaser und die Phasenverzögerungsplatte für eine Korrektur der Polarisation in einem freien Strahlengang genutzt wird. Diese Bewegung der Paddles, also optischer "Paddel", erfolgt also in Abhängigkeit von der Bewegung des Systems, und zwar derart, dass die Änderung der Polarisation, in der Regel also eine Polarisationsdrehung, aufgehoben und (ungefähr) in den ursprüng-glichen Zustand zurückversetzt wird.

Eine freie Leitung des Untersuchungsstrahls ist hierbei insbesondere eine Leitung bzw. Führung des Untersuchungsstrahls mittels Linsen, Spiegel, etc. also nicht im Inneren einer Lichtleitfaser, sondern über einen freien optischen Weg.

Die Messung der Änderung der Polarisation des Untersuchungsstahls erfolgt, wie oben schon erwähnt, beispielsweise mittels einer Referenzanordnung, die einen Strahlteiler im Strahlengang hinter einem Kollimator, der am Ausgang aus einer Lichtleitfaser angeordnet ist, und eine Photodiode hinter einem Polarisator enthält: Der Strahlteiler lenkt somit einen Teil der Strahlung auf die Photodiode, mit der die Polarisation analysiert werden kann.

Die Änderung der internen Lagebeziehungsänderung von Komponenten des Systems kann durch eine externe Bewegung, wie beispielsweise bewegliche Arme des Systems, getriggert sein. Andererseits gibt es auch reine interne Änderungen der Lagebeziehung von Komponenten, wie beispielsweise die bereits beschriebene laterale Bewegung der Linse zur Erweiterung des Bildfeldes. Komponenten des Systems sind hier jegliche optische Komponenten wie beispielsweise Linsen und Linsensysteme, verstellbare wie feste Spiegel, optische Freiformflächen, Lichtleitfasern oder andere Strahlführungskomponenten.

Eine Änderung der internen Lagebeziehung kann des Weiteren aus ganz anderem Grunde erfolgen, nämlich beim Austausch von Komponenten des Systems, die nicht in exakt derselben Größe und Lage eingesetzt werden können. Auch dann ist eine - in der Regel einmalige - Korrektur erforderlich, wenn nach dem Austausch von Komponenten eine direkte Vergleichbarkeit zu Untersuchungen gewährleistet werden soll, die vor dem Austausch der entsprechenden Komponenten erfolgte.

In einer weiteren besonderen Ausführungsform des Verfahrens zur direkten Korrektur einer Abbildung wird der Untersuchungsstrahl frei durch ein in seiner internen Lagebeziehung seiner Komponenten variierbares System geleitet, und eine während einer Lagebeziehungsänderung des Systems entstehende Änderung der optischen Weglänge durch eine variierbare Zusatzfaser oder eine Delay Line korrigiert. Mittels einer solchen variierbaren Zusatzfaser bzw. Delay Line wird die entstandene Änderung der optischen Weglänge des Untersuchungsstrahls wieder ausgeglichen, so dass auch hier bezüglich der optischen Weglänge des Untersuchungsstrahls ein ursprünglicher Zustand (wie ohne Variation der Lagebeziehung der Komponenten des Systems) wiederhergestellt werden kann.

In einer bevorzugten Ausführungsform des Verfahrens zur Korrektur einer Abbildung wird der Untersuchungsstrahl frei durch ein in seiner internen Lagebeziehung seiner Komponenten variierbares System geleitet. Während dieser Lagebeziehungsänderung des Systems ändert sich die optische Weglänge des Untersuchungsstrahls und beeinflusst damit die Abbildung bzw. die Bilddaten der Abbildung. In Korrelation zur Änderung der optischen Weglänge des Untersuchungsstrahls und somit als "Effekt 0. Ordnung" entsteht dabei eine Scherung der Abbildung der optischen Kohärenztomographie, die als Verzerrung der Abbildung wahrgenommen wird. Um diesem Effekt entgegenzuwirken, wird die Abbildung bzw. werden die Bilddaten der Abbildung in dieser Ausführungsform in Abhängigkeit von der Änderung der optischen Weglänge direkt, d.h. auf rechnerischem Wege, korrigiert.

Es ist dabei auch möglich, sowohl ein verstellbares Korrekturelement zur partiellen Korrektur einer Änderung der optischen Weglänge des Untersuchungsstrahls einzusetzen und einen weiteren Teil einer Änderung der optischen Weglänge direkt, also auf rechnerischem Wege, zu korrigieren. Die physikalische Korrektur durch den Einsatz von verstellbaren Korrekturelementen und die direkte, rechnerische Korrektur sind also miteinander kombinierbar.

Ändert sich eine optische Weglänge des Untersuchungsstrahls kontinuierlich, gleichförmig oder nicht gleichförmig, so sollte zudem die Geschwindigkeit der Änderung der optischen Weglänge (dOPD/dt) des Untersuchungsstrahls berücksichtigt werden. In einer weiteren bevorzugten Ausführungsform des Verfahrens zur Korrektur der Abbildung, insbesondere einer Abbildung für die ein "Time-Domain" (TD)-OCT- oder "Swept Source" (SS)-OCT-Verfahren genutzt wird, wird eine Verschiebung der Abbildung in Abhängigkeit von der Geschwindigkeit der Änderung der optischen Weglänge (dOPD/dt) des Untersuchungsstrahls direkt korrigiert. Diese zusätzliche Verschiebung der Abbildung in Abhängigkeit von der Geschwindigkeit der Änderung der optischen Weglänge ist ein "Effekt 1. Ordnung", der in seinen Auswirkungen auf die Abbildung in der Regel zwar wesentlich weniger stark als der oben beschriebene "Effekt 0. Ordnung", aber dennoch signifikant ist. Für eine optimale Korrektur der Abbildung sollten also sowohl der "Effekt 0. Ordnung" als auch der "Effekt 1. Ordnung" berücksichtigt werden.

Des Weiteren ist es vorteilhaft, wenn in einem Verfahren zur direkten Korrektur der Abbildung, die auf einer SS-OCT-Untersuchung basiert, ein Sweep-Bereich definiert wird, bei dem ein Auflösungsoptimum der Abbildung erreicht wird. Im Gegensatz zu statischen OCT-Messungen verbreitert sich die Peakbreite aufgrund der Änderung der optischen Weglänge, auch wenn die Sweep-Geschwindigkeit als konstant angenommen wird, was wiederum zu einer Verringerung der Auflösung führt.

Ein ophthalmologisches Untersuchungssystem umfasst eine Vorrichtung für die optische Kohärenztomographie(OCT). Es umfasst hierfür eine Vorrichtung zur Erzeugung eines Untersuchungsstrahls, also eine Strahlungsquelle, insbesondere eine Laserquelle. Des Weiteren umfasst es eine Analysevorrichtung für die Analyse des von einer Struktur eines Targets zurückgesendeten Untersuchungsstrahls und zur Erzeugung von Bilddaten einer Abbildung der Struktur des Targets.

Das ophthalmologische Untersuchungssystem kann noch weitere Untersuchungsvorrichtungen umfassen bzw. kann auch Teil eines ophthalmologischen Therapiesystems, insbesondere Teil eines Systems zur Augenchirurgie, sein.

Eine Vorrichtung für die optische Kohärenztomographie(OCT) enthält in ihrem prinzipiellen Aufbau einen sogenannten "Signalarm", durch den der Untersuchungsstrahl verläuft und einen "Referenzarm", durch den der Referenzstrahl verläuft. Der Strahl, der von der Strahlungsquelle erzeugt wird, wird durch einen Strahlteiler in Untersuchungsstrahl und Referenzstrahl geteilt. Nach ihren Wegen - der Untersuchungsstrahl zum Target und wieder zurück, der Referenzstrahl im "Referenzarm" - wird in der Analysevorrichtung der Untersuchungsstrahl mit dem Referenzstrahl verglichen. Untersuchungsstrahl und Referenzstrahl werden hierzu vor der Analysevorrichtung zur Interferenz gebracht und diese beispielsweise mit einem klassischen Detektor in der Analysevorrichtung analysiert. Die Analysevorrichtung erzeugt daraus Bilddaten einer Abbildung der Struktur eines Targets, mit denen dann ggf. auf einer Anzeigevorrichtung bzw. einem Bildschirm eine Abbildung erzeugt werden kann.

Das ophthalmologische Untersuchungssystem enthält nun des Weiteren eine Strahlführungsvorrichtung zur Führung des Untersuchungsstrahls von der Vorrichtung zur Erzeugung des Untersuchungsstrahls zum Target und vom Target zur Analysevorrichtung. Eine solche Strahlführungsvorrichtung kann allein zur Führung des Untersuchungsstrahls einer Vorrichtung für die optische Kohärenztomographie genutzt werden oder aber auch gleichzeitig oder sequentiell zur Führung anderer Strahlung genutzt, also zumindest über eine Teilstrecke auch die Strahlung anderer Systeme des ophthalmologischen Untersuchungssystems führen - sequenziell oder gleichzeitig. Insbesondere kann die Strahlführungsvorrichtung auch für die Führung des Therapiestrahls genutzt werden.

Die Strahlführungsvorrichtung kann zur Führung der Strahlung Scanner, bewegliche oder feste Spiegel, bewegliche oder feste Linsen oder andere optische Elemente, die einen Untersuchungsstrahl in Ihrer Richtung und/oder Ihrer Fokussierung verändern, enthalten. Die Strahlführungsvorrichtung erstreckt sich dabei von der Vorrichtung zur Erzeugung eines Untersuchungsstrahls über den Signalarm bis zur Analysevorrichtung.

Das zu untersuchende Target befindet sich in einem Untersuchungsvolumen, das einen Bereich des Auges umfassen kann und eine definierte räumliche Ausdehnung in allen drei Raumrichtungen aufweist. Das Target weist Strukturen auf, die durch die Untersuchung genau bestimmt werden sollen.

Im vorliegenden ophthalmologischen Untersuchungssystem sind dabei eine optische Weglänge und/oder eine Polarisation des Untersuchungsstrahls in der Strahlführungsvorrichtung diskret oder kontinuierlich veränderlich. Ursächlich hierfür ist z.B. ein in der internen Lagebeziehung seiner Komponenten variierbares Untersuchungssystem. Insbesondere ist eine bewegliche Objektivlinse und/oder bewegliche Gelenkarme, durch die der Untersuchungsstrahl hindurchgeführt wird und/oder ein Austausch von Komponenten des ophthalmologischen Untersuchungssystems Ursache für die Änderung der optischen Weglänge und/oder der Polarisation des Untersuchungsstrahls. Eine solche Änderung ist diskret, wenn sie einmalig (z.B. im Fall des Austauschs von Komponenten), oder mehrfach durch diskrete Einstellungen (z.B. Positionseinstellungen der Arme bzw. insbesondere der beweglichen Objektivlinse) erfolgt, oder aber kontinuierlich, wenn sich die optische Weglänge beständig ändert (z.B. durch kontinuierliche Bewegung der beweglichen Objektivlinse, wobei letzteres nicht nur zwischen zwei A-Scans sondern auch während der Durchführung eines A-Scans des OCTs die optische Weglänge ändert).

Das ophthalmologische Untersuchungssystem enthält weiterhin eine Steuervorrichtung zu seiner Steuerung, wobei die Steuervorrichtung einteilig ausgeführt sein kann oder aber mehrere auch räumlich voneinander getrennte Steuereinheiten umfassen kann.

Erfindungsgemäß ist nun die diskrete oder kontinuierliche Veränderung der optischen Weglänge und/oder der Polarisation des Untersuchungsstrahls in Abhängigkeit von der Zeit bekannt oder mittels einer Referenzanordnung messbar.

Das erfindungsgemäße ophthalmologische Untersuchungssystem enthält weiterhin ein verstellbares Korrekturelement zum Ausgleich der Änderung der optischen Weglänge und/oder der Polarisation Gleichzeitig oder alternativ weist die Steuervorrichtung eine Korrektureinheit auf, die eingerichtet ist zur direkten Korrektur der Abbildung oder der Bilddaten der Abbildung in Abhängigkeit von der Änderung der optischen Weglänge des Untersuchungsstrahls und/oder der Polarisation in der Strahlführungsvorrichtung.

Diese Korrektureinheit ist also kodiert, um eine direkte Korrektur der Abbildung oder der Bilddaten der Abbildung in Abhängigkeit von der Änderung der optischen Weglänge und/oder der Polarisation des Untersuchungsstrahls durchzuführen. Die Korrektur erfolgt derart, dass der Einfluss der Änderung der zurückgelegten Weglänge des Untersuchungsstrahls zum und/oder vom Target und/oder der Polarisation des Untersuchungsstrahls auf die Abbildung bzw. die Bilddaten der Abbildung ausgeglichen wird, also die Modifizierung der Abbildung bzw. der Bilddaten der Abbildung direkt korrigiert wird. Für die Polarisation ist jedoch eine Korrektur durch ein verstellbares Korrekturelement bei Weitem einer rechnerischen Nachbearbeitung bevorzugt.

Bevorzugt enthält das ophthalmologische Untersuchungssystem weiterhin eine Anzeigevorrichtung zur Sichtbarmachung der Abbildung mit Hilfe der Bilddaten der Abbildung einer Struktur des Targets. Eine solche Anzeigevorrichtung ist in der Regel ein Bildschirm, auf dem die entsprechend untersuchte Struktur des Targets abgebildet wird. Aber auch eine reine Anzeige der Bilddaten ist möglich. Eine solche Anzeigevorrichtung kann aber muss nicht räumlich an oder in dem ophthalmologischen Untersuchungssystem enthalten sein. Eine Anzeigevorrichtung, die in räumlicher Entfernung zum ophthalmologischen Untersuchungssystem mit diesem über eine Datenverbindung kommuniziert ist ebenfalls möglich. Alternativ kann ein ophthalmologisches Untersuchungssystem ganz ohne Anzeigevorrichtung auskommen, wenn die Bilddaten der Abbildung gespeichert und auf einem unabhängigen System sichtbar gemacht werden.

In einer Ausführungsform des ophthalmologischen Untersuchungssystems weist die Strahlführungsvorrichtung ein in seiner internen Lagebeziehung seiner Komponenten variierbares System auf und enthält eine Lichtleitfaser. Mindestens ein Teil der Strahlführungsvorrichtung verläuft also beispielsweise durch einen beweglichen Gelenkarm hindurch. Eine Leitung durch eine Lichtleitfaser führt zu einer im Prinzip konstanten optischen Weglänge (für die Strecke des optischen Weges, der durch die Lichtleitfaser hindurch verläuft, wobei temperaturabhängige Änderungen der optischen Weglänge in der Lichtleitfaser bei Temperaturschwankungen selbstverständlich auftreten können, aber entsprechend berücksichtigt werden), allerdings ist eine Polarisationsdrehung aufgrund einer Bewegung der Lichtleitfaser, beispielsweise durch eine Bewegung eines Gelenkarms, durch den die Lichtleitfaser hindurchgeführt wird, möglich.

Unter dem Begriff Lichtleitfaser soll dabei jegliche Art von optischer Faser verstanden werden, durch die ein Untersuchungsstrahl ohne größere Verluste hindurchgeleitet werden kann.

In einer alternativen Ausführungsform oder aber in Fortführung der oben beschriebenen Ausführungsform des ophthalmologischen Untersuchungssystems weist die Strahlführungsvorrichtung ein in seiner internen Lagebeziehung seiner Komponenten variierbares System auf, und mindestens ein Teil der Strahlführungsvorrichtung ist eingerichtet, den Untersuchungsstrahl frei durch das in seiner internen Lagebeziehung seiner Komponenten variierbare System zu führen.

Einen Untersuchungsstrahl frei zu führen heißt hier, dass die Strahlführung nicht über eine Lichtleitfaser, sondern mittels optischer Komponenten wie feste oder bewegliche Spiegel, feste oder bewegliche Linsen etc. erfolgt, der optische Weg also frei verläuft. Dies führt ggf. zu Änderungen des optischen Weges (wenn sich beispielsweise die Lage zweier optischer Komponenten zueinander ändert) und/oder der Polarisation.

In einer bevorzugten Ausführungsform des ophthalmologischen Untersuchungssystems umfasst das verstellbare Korrekturelement, das zum direkten Ausgleich eingesetzt wird, zwei bewegliche Paddles, die sich in Abhängigkeit von der Bewegung des Systems mitbewegen, oder eine Phasenverzögerungsplatte zum Ausgleich der Änderung der Polarisation des Untersuchungsstrahls und/oder eine variierbare Zusatzfaser oder eine Delay Line zum Ausgleich der Änderung der optischen Weglänge des Untersuchungsstrahls. Das verstellbare Korrekturelement ist, bevorzugt in einer der genannten Ausgestaltungen oder in einer Kombination dieser Ausgestaltungen, in der Strahlführungsvorrichtung des Untersuchungsstrahls und/oder in einer alternativen Strahlführungsvorrichtung für einen Referenzstrahl enthalten.

Der Referenzstrahl ist hierbei der Strahl, der in einer alternativen Strahlführungsvorrichtung des Referenzarms verläuft und in der Analysevorrichtung zum Vergleich durch Interferenz mit dem Untersuchungsstrahl genutzt wird, um hieraus die Abbildung der Strukturen des Targets, zu dem der Untersuchungsstrahl verlaufen ist bzw. die Bilddaten dieser Abbildung zu erzeugen.

Bevorzugt ist also ein solches Korrekturelement nur in der Strahlführungsvorrichtung des Untersuchungsstrahls enthalten, so dass dort direkt der Änderung der optischen Weglänge und/oder der Polarisation entgegengewirkt werden kann. Ist das Korrekturelement jedoch in der alternativen Strahlführungsvorrichtung für den Referenzstrahl enthalten und nicht in der Strahlführungsvorrichtung des Untersuchungsstrahls, dann muss das Korrekturelement eine gleichwirkende Änderung bewirken wie die vorliegende diskrete oder kontinuierliche Veränderung der optischen Weglänge und/oder der Polarisation des Untersuchungsstrahls. Letztere Variante der Positionierung des Korrekturelements ist dabei fehlerbehafteter als die Positionierung eines Korrekturelements im der Strahlführungsvorrichtung des Untersuchungsstrahls, da durch den räumlich unterschiedlichen Verlauf dieser alternativen Strahlführungsvorrichtung an anderer Stelle als am Ort des Entstehens korrigiert wird.

In einer anderen bevorzugten Ausführungsform des ophthalmologischen Untersuchungssystems ist eine Korrektureinheit der Steuervorrichtung eingerichtet, eine in Abhängigkeit von der Änderung der optischen Weglänge auftretende Scherung der optischen Kohärenztomographie-Abbildung, also den "Effekt 0. Ordnung", der direkt zur Änderung der optischen Weglänge des Untersuchungsstrahls korreliert, zu korrigieren. Dies kann beispielsweise derart erfolgen, dass die Bilddaten der Abbildung direkt mit einem Korrekturwert, der von der Änderung der optischen Weglänge zum jeweiligen Zeitpunkt abhängig ist, beaufschlagt werden.

In einer Ausführungsform des ophthalmologischen Untersuchungssystems, in der eine Korrektureinheit der Steuervorrichtung direkte Korrekturen der Abbildung bzw. der Bilddaten der Abbildung vornimmt, und sich eine optische Weglänge des Untersuchungsstrahls kontinuierlich ändert, wird vorzugsweise auch die Geschwindigkeit der Änderung der optischen Weglänge (dOPD/dt) des Untersuchungsstrahls berücksichtigt: Umfasst eine Vorrichtung für die optische Kohärenztomographie eines ophthalmologischen Untersuchungssystems also insbesondere eine Vorrichtung für "Time-Domain" (TD)-OCT oder "Swept Source" (SS)-OCT, so ist dessen Korrektureinheit auch eingerichtet, eine Verschiebung der optischen Kohärenztomographie-Abbildung in Abhängigkeit von der Geschwindigkeit der Änderung der optischen Weglänge (dOPD/dt), also den "Effekt 1. Ordnung", zu korrigieren.

Bevorzugt erfolgt also, insbesondere bei OCT-Versionen oder anderen Untersuchungsmethoden, für die die durchlaufene optische Weglänge und/oder Polarisation eine Rolle spielt, und bei denen die Änderung der optischen Weglänge erfolgt, die Korrektur der Abbildung oder der Bilddaten der Abbildung sowohl bezüglich des Effekts "0. Ordnung" als auch bezüglich des "Effekts 1. Ordnung".

In einer bevorzugten Ausführungsform enthält die Strahlführungsvorrichtung des Untersuchungsstrahls des ophthalmologischen Untersuchungssystems eine bewegliche Optik, insbesondere eine lateral bewegliche Objektivlinse. Eine lateral bewegliche Objektivlinse ist demnach senkrecht einer optischen Achse z in einer x-y Ebene beweglich. Eine Strahlführung kann dann realisiert werden mit Hilfe von ebenfalls beweglichen Umlenkelementen, wie beweglichen und verkippbaren Spiegeln, die der Bewegung der Objektivlinse folgen. Diese Bewegung der Objektivlinse erfolgt mit dem Ziel, ein Gesamtbildfeld zu erreichen, das in seiner Ausdehnung einem Vielfachen des Bildfeldes der Objektivlinse entspricht.

Die bewegliche Optik kann dabei auch durch mehrere bewegliche Linsen realisiert werden, die eine laterale Beweglichkeit aufweisen, aber zudem auch eine Beweglichkeit parallel zur optischen Achse aufweisen können.

Sowohl die bewegliche Optik als auch die Umlenkelemente können in mehr als einer lateralen Richtung beweglich ausgebildet sein.

Weiterhin ist es von Vorteil, wenn das ophthalmologische Untersuchungssystem eine Vorrichtung für die optische Kohärenztomographie (OCT) aufweist, die in abgeschlossene, unabhängig voneinander austauschbare und einzeln korrigierbare Teilsysteme mit definierten Übergängen unterteilt ist, bevorzugt jedoch in ein erstes Teilsystem, das ein Interferometer enthält und ein zweites Teilsystem, das eine Lichtleitfaser mit einem Kollimator umfasst.

Alternativ erfolgt die Aufteilung in ein erstes Teilsystem, das ein Interferometer enthält, ein zweites Teilsystem, das eine lange Lichtleitfaser enthält, die durch ein in seiner internen Lagebeziehung seiner Komponenten variierbares System hindurch verläuft, und in ein drittes Teilsystem, dass eine kurze Lichtleitfaser und einen Kollimator umfasst. Eine solche Unterteilung der Vorrichtung für die optische Kohärenztomographie erleichtert einen Austausch einzelner Teile dieser Vorrichtung wesentlich: Ein Korrekturelement, aber auch eine Korrektur mittels der Korrektureinheit der Steuervorrichtung ist dann sehr einfach und in bekannter Weise einsetzbar.

Für die Produktion und den späteren Service ist es von großem Vorteil, das ophthalmologische Untersuchungssystem in verschiedene korrigierbare Teilsysteme aufzuteilen, deren Beziehung zueinander leicht bestimmbar ist. Eine Aufteilung eines Untersuchungssystems, insbesondere eines ophthalmologischen Untersuchungssystems, das bewegliche Gelenkarme umfasst, und das nach den Prinzipien der optischen Kohärenztomographie arbeitet, erfolgt dabei also in mindestens zwei oder drei Teilsysteme wie oben beschrieben. Aber auch andere Aufteilungen sind denkbar - vorausgesetzt, dass sie definierte Schnittstellen erzeugen.

Eine solche Aufteilung des ophthalmologischen Untersuchungssystems in Teilsysteme, die leicht nach den hier genannten Prinzipien korrigierbar sind, ermöglicht es, einzelne Komponenten zu tauschen. Um die optische Weglänge in dem beweglichen Gelenkarm, über den der Untersuchungsstrahl geführt wird, auch bei einem Tausch einer Subkomponente zu gewährleisten, wird zusätzlich eine Möglichkeit der Korrektur der optischen Weglängenänderung eingefügt, bei Nutzung eines Untersuchungssystems, das nach den Prinzipien der optischen Kohärenztomographie arbeitet, entweder in das entsprechende Teilsystem oder in einen Referenzarm. Dies kann beispielsweise eine variierbare Zusatzfaser sein, die in verschiedenen Längen verfügbar ist, oder aber eine Delay Line - also ein verstellbares Korrekturelement, das in diesem Fall einmalig nach dem Service am Untersuchungssystem, bei dem eine Subkomponente ausgetauscht wurde, verstellt wird.

Wird die variierbare Zusatzfaser bzw. die Delay Line im Referenzarm eingebaut, so wird das Signal-Rausch-Verhältnis weniger beeinflusst als beim Einbau in das entsprechende Teilsystem des Signalarmes.

Der Kollimator kann zusätzlich in einer kleinen Röhre gehalten werden, um durch variables Einkleben des Kollimators in das Röhrchen eine gleiche optische Weglänge vom Fasereingang bis zum Ende des Metallröhrchens für alle Lichtleitfasern mit Kollimatoren zu erreichen. Damit soll gewährleistet werden, dass Varianzen in den Faserlängen durch das variable Einkleben ausgeglichen werden können. Durch Fertigungstoleranzen ist die Faserlänge vom Fasereingang bis zum Kollimator nicht exakt gleich. Durch das zusätzliche Röhrchen kann die optische Weglänge künstlich gleich gemacht werden, indem man für manche Fasern mehr, für andere weniger Luftweg bis zum Röhrchenende hinzu gibt. Dieses Prinzip ist eine Alternative zu einer zusätzlichen Delay Line, es kann aber auch in Kombination mit einer Delay Line verwendet werden.

Auch ist es von Vorteil, wenn ein ophthalmologisches Untersuchungssystem zudem eine Therapielaservorrichtung umfasst, wobei entweder eine Vorrichtung zur Erzeugung des Untersuchungsstrahls ebenfalls zur Erzeugung des Therapielaserstrahls eingerichtet ist oder eine unabhängige Vorrichtung zur Erzeugung des Therapielaserstrahls neben der Vorrichtung zur Erzeugung des Untersuchungsstrahls enthalten ist. Im ersten Fall ist die Energie der Laserstrahlung, die sowohl für die Untersuchung mit einer ersten Energie als auch für die Lasertherapie mit einer zweiten, deutlich höheren Energie eingesetzt wird, entsprechend über einen größeren Energiebereich regelbar. Wird für die Erzeugung des Untersuchungsstrahls als auch für die Erzeugung des Therapielaserstrahls jeweils eine unabhängige Vorrichtung genutzt, so können die jeweiligen Erzeugungsvorrichtungen einfacher auf die jeweiligen Bedürfnisse angepasst werden. Eine bevorzugte Vorrichtung zur Erzeugung des Therapielaserstrahls umfasst eine Kurzpuls-Laserquelle, insbesondere eine Femtosekunden- oder eine Pikosekunden-Laserquelle.

Eine Korrektureinheit für eine Steuervorrichtung eines ophthalmologischen Untersuchungssystems, das eine Vorrichtung für die optische Kohärenz-Tomographie (OCT) umfasst, ist eingerichtet, eine Abbildung einer Struktur eines Targets, die auf einer Untersuchung des Targets mit einem Untersuchungsstrahl einer Vorrichtung für die optische Kohärenztomographie (OCT) basiert, und bei deren Erzeugung eine zurückgelegte optische Weglänge von einer Quelle des Untersuchungsstrahls zu der Struktur des Targets und/oder von der Struktur des Targets zu einer Analysevorrichtung und/oder eine Polarisation des Untersuchungsstrahls ausgewertet wird, wobei sich die optische Weglänge des Untersuchungsstrahls zum und/oder vom Target und/oder ihre Polarisation im Verlaufe der Erzeugung der Abbildung der Struktur des Targets diskret oder kontinuierlich ändert, und die Änderung der optischen Weglänge des Untersuchungsstrahls zum und/oder vom Target und/oder die Änderung ihrer Polarisation bekannt ist oder gemessen wird, derart zu korrigieren, dass die Modifizierung der Abbildung durch die bekannte oder gemessene Änderung der optischen Weglänge und/oder der Polarisation des Untersuchungsstrahls korrigiert wird.

Bezüglich Target, Material im Targetbereich und Änderung der optischen Weglänge des Untersuchungsstrahls zum Target und/oder Änderung der Polarisation des Untersuchungsstrahls gilt das oben gesagte. Auch bezüglich dessen, in welcher Weise eine Änderung der optischen Weglänge und/oder der Polarisation des Untersuchungsstrahls bekannt sein kann oder gemessen werden kann wird auf die oben gemachten Ausführungen verwiesen.

Ursachen für eine Änderung der optischen Weglänge und/oder der Polarisation des Untersuchungsstrahls, deren Einflüsse auf eine Abbildung der Struktur mit einer solchen Korrektureinheit behoben werden können, sind - wie oben schon erwähnt - vielzählig. Zu den Ursachen gehören bewegliche Komponenten eines entsprechenden Untersuchungssystems, das der Untersuchungsstrahl durchläuft, wie bewegliche Linsen, insbesondere eine bewegliche Objektivlinse mit dafür notwendigen Ablenkelementen wie Spiegel, ein extern bewegliches System, insbesondere ein Untersuchungssystem, das bewegliche Gelenkarme aufweist, über die ein Untersuchungsstrahl zu einem Target geführt wird, aber auch Änderungen bzw. ein Austausch von Teilen des Untersuchungssystems, die für die Strahlführung verantwortlich sind.

Für die meisten Ursachen kann die Änderung der optischen Weglänge und/oder der Polarisation In Abhängigkeit von der Zeit und/oder der Position von Komponenten des Systems beschrieben werden.

In einer Variante der Korrektureinheit wird eine durch eine Änderung der optischen Weglänge entstehende Scherung der Abbildung in Abhängigkeit von der Änderung der optischen Weglänge korrigiert. Hierbei handelt es sich um die Korrektur des "Effekts 0. Ordnung", also die Korrektur der Verkippung bzw. Scherung aufgrund der Änderung der optischen Weglänge In Abhängigkeit von der aktuellen optischen Weglänge.

In einer Variante der Korrektureinheit wird zusätzlich eine Verschiebung der Abbildung in Abhängigkeit von der Geschwindigkeit der Änderung der optischen Weglänge (dOPD/dt) korrigiert. Diese zusätzliche Korrektur des "Effekts 1. Ordnung" erfolgt also in Abhängigkeit von der (ggf. variablen) Geschwindigkeit der Änderung der optischen Weglänge.

Wird ein SS-OCT genutzt, erfolgt diese Korrektur auch in Abhängigkeit von der Frequenz der OCT-Strahlung.

Eine Korrektureinheit für eine Steuervorrichtung einer Vorrichtung für Swept-Source OCT ist eingerichtet, einen Sweep-Bereich zu definieren, bei dem ein Auflösungsoptimum der Abbildung erreicht wird.

In Abhängigkeit von der Sweep-Geschwindigkeit wird ein Sweep-Bereich definiert, weil im Vergleich zu einer klassischen, statischen OCT-Messung, bei der keine Änderung der optischen Weglänge des Untersuchungsstrahls erfolgt, insbesondere bei einer Variation der Geschwindigkeit der Änderung der optischen Weglänge die Auflösung aufgrund einer Verbreiterung der Peakbreite verringert wird.

Die Prinzipien des erfindungsgemäßen Verfahrens zur Korrektur einer Abbildung, die auf einer Untersuchung mittels optische Kohärenztomographie(OCT) basiert und des erfindungsgemäßen Untersuchungssystems für Untersuchungen, während denen sich eine optische Weglänge und/oder die Polarisation des Untersuchungsstrahls ändert, seien an dieser Stelle nun auf die konkrete Anwendung der Swept Source (SS)-OCT Untersuchung konkretisiert:
Wird ein ophthalmologisches Untersuchungssystem genutzt, das bewegte Gelenkarme enthält, über die der Untersuchungsstrahl zum Auge eines Patienten geführt wird, und wird dabei der Untersuchungsstrahl eines SS-OCT-Moduls mittels einer Lichtleitfaser durch einen Gelenkarm geleitet, so ändert sich durch die Faserverlegung in dem beweglichen Gelenkarm die Ausgangspolarisation in einem Applikator am Ende des Gelenkarms bei einer Bewegung des Gelenkarms. Um die Polarisationsdrehung auszugleichen sind zwei motorisierte Paddles in einem OCT-Modul verbaut, die jede gewünschte Ausgangspolarisation wieder einstellen können.

Wie oben beschrieben, ist im Applikator am Ende des Gelenkarms ein Analysator verbaut der in Kombination mit einer Photodiode die Polarisation bestimmen kann. Nach einer Armbewegung wird die Polarisation mit Hilfe der Paddles wieder eingestellt und ein optimales Signal-Rausch-Verhältnis ist gewährleistet. Ohne die Korrektur wäre das Signal-Rauschverhältnis schlechter oder es muss ein komplexer Algorithmus angewandt werden, um das Signal Rausch Verhältnis anders zu optimieren.

Wird ein SS-OCT in Kombination mit einer bewegten Optik des ophthalmologischen Untersuchungssystems genutzt, so bewegt sich die Optik, während die einzelnen OCT-Scans aufgenommen werden. Damit ändert sich die optische Weglänge zum Target.

Dies führt erstens zu einem Offset, der direkt mit dieser Änderung korreliert. Diese Änderung kann direkt in die Abbildung eingerechnet werden und die Abbildung damit entzerrt und somit korrigiert werden.

Da die Bewegung auch während eines A-Scans erfolgt, ergibt sich weiterhin auch eine Verschiebung ("Shift") der Abbildung in Abhängigkeit von der aktuellen Geschwindigkeit der bewegten Optik, präziser der aktuellen Geschwindigkeit der Änderung der optischen Weglänge, inklusive dessen Vorzeichen. Hierzu wird an jedem A-Scan die Geschwindigkeit der optischen Weglängenänderung berechnet und über diese und die Scan-Geschwindigkeit der Swept Source die jeweilige Zusatzkorrektur berechnet. Auf diese Weise ist es möglich einen korrekten B-Scan zu ermitteln, obwohl sich während der einzelnen A-Scans die optische Weglänge aktiv ändert.

Alternativ kann ein Untersuchungsstrahl eines SS-OCT in einem ophthalmologischen Untersuchungssystem, das bewegliche Gelenkarme enthält, über die der Untersuchungsstrahl auf das Auge eines Patienten geführt wird, auch frei durch den Gelenkarm geführt werden. Insbesondere, wenn das ophthalmologische Untersuchungssystem auch eine Therapielaservorrichtung umfasst, und somit auch ein Therapielaserstrahl über einen beweglichen Gelenkarm zum Auge des Patienten geführt wird, können der Therapielaserstrahl und der Untersuchungsstrahl des SS-OCT zusammen durch den beweglichen Gelenkarm geführt werden.

Eine freie Führung des Untersuchungsstrahls durch einen beweglichen Gelenkarm erfordert gute antireflektive Schichten auf den Optiken, um nicht zu viele Reflexe zu erhalten, sowie ggf. Spezialschichten für die Wellenlänge des Untersuchungsstrahls, um die Polarisation zu halten. Im Falle einer Änderung der Polarisation kann diese aber genauso über bewegliche Paddles korrigiert werden. Falls der Phasendrift über der Wellenlänge sehr stark ist, kann dann das optimale Signal-Rausch-Verhältnis allerdings nur noch für eine Wellenlänge eingestellt werden.

Lösungen zur entsprechenden Korrektur einer Abbildung, insbesondere bei Nutzung der optischen Kohärenztomographie in einem ophthalmologischen Untersuchungssystem, bedarf es ebenso, wenn sich die optische Weglänge und/oder die Polarisation im Rahmen von Service-Maßnahmen ändern. Dies erfolgt, wie oben bereits beschrieben.

Die vorliegende Erfindung soll nun anhand von Ausführungsbeispielen erläutert werden. Es zeigt:

- die Fig. 1 eine Ausführungsform eines ophthalmologischen Untersuchungssystems;
- die Fig. 2 ein optisches System mit Vorrichtungen zur Erzeugung eines Untersuchungsstrahls bzw. eines Therapiestrahls und Strahlführungsvorrichtung, das in einem ophthalmologischen Untersuchungssystem gemäß Fig. 1 eingesetzt werden kann;
- die Fig. 3a bis 3d: den Einfluss einer beweglichen Objektivlinse auf die optischen Weglänge eines Untersuchungsstrahls in einer Strahlführungsvorrichtung eines ophthalmologischen Untersuchungssystems;
- die Fig. 4: Die Änderung der optischen Weglänge eines Untersuchungsstrahls, insbesondere eines OCT-Untersuchungsstrahls, bei Einsatz einer beweglichen Objektivlinse;

Fig. 5a bis 5c: eine schematisierte Abbildung einer Cornea und einer Linse ohne Modifizierung der Abbildung, also nicht verzerrt und in der richtigen Position; mit Scherung durch den Einfluss der Änderung der optischen Weglänge; und zusätzlich verschoben durch den Einfluss der Geschwindigkeit der Änderung der optischen Weglänge bei Einsatz eines OCT-Linienscans (B-Scan als Aneinanderreihung vieler A-Scans);

Fig. 6a und 6b: eine schematisierte Abbildung einer Cornea und einer Linse nicht verzerrt und in der richtigen Position; mit Scherung durch den Einfluss der Änderung der optischen Weglänge; und zusätzlich verschoben durch den Einfluss der Geschwindigkeit der Änderung der optischen Weglänge für ein Chirped OCT mit Kreisscan;

Fig. 7: den Einfluss der z-Geschwindigkeit auf ein swept-Source OCT-Signal;

Fig. 8: Die Abhängigkeit der Auflösung der optischen Weglänge, insbesondere des Auflösungsoptimums, vom Durchstimmbereich;

Fig. 9: Ein OCT-Modul eines ophthalmologischen Untersuchungssystems.

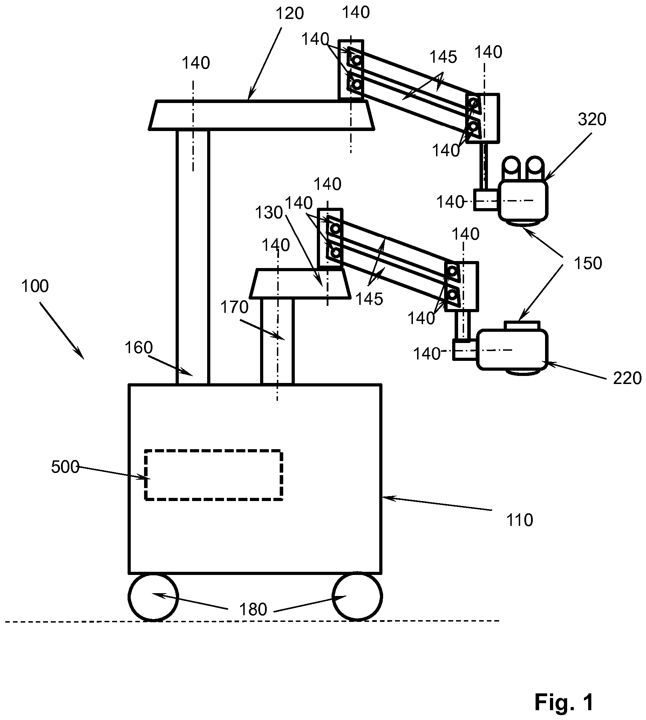

In der Fig. 1 ist ein Ausführungsbeispiel eines erfindungsgemäßen ophthalmologischen Untersuchungssystems 100, das eine Therapielaservorrichtung umfasst - in diesem Falle also genau genommen ein ophthalmologisches Untersuchungs- und Therapiesystem 100 - in einer Seitenansicht dargestellt. Dieses ophthalmologische Untersuchungssystem 100 enthält einen Grundkörper 110, der auf einer Bewegungseinrichtung 180, die Rollen aufweist, gelagert ist. Aus diesem Grundkörper 110 heraus ragen zwei als Achsen wirkende Stützstrukturen 160, 170 an denen jeweils ein Gelenkarm 120, 130 angeordnet ist. Der erste Gelenkarm 120 enthält ein Operationsmikroskop-Kopf 320, während am zweiten Gelenkarm 130 ein Laser-Applikator-Kopf 220 angeordnet ist, aus dem im Betrieb eine fokussierte, gepulste Laserstrahlung, die in einer im Grundkörper 110 befindlichen (hier nicht gezeigten) Laserquelle - in diesem Fall eines Femtosekunden-Lasers - erzeugt wird und über eine Strahlführungsvorrichtung in Stützstruktur 170 und Gelenkarm 130 und eine Strahlfokussierungsoptik als Teil der Strahlführungsvorrichtung zum Laser-Applikator-Kopf 220 geleitet wird. Beide Gelenkarme 120, 130 sind über ihre Gelenke 140 nahezu beliebig im Raum beweglich. Alle Komponenten dieses ophthalmologischen Untersuchungssystems 100 werden durch eine Steuervorrichtung 500 gesteuert.

Die beiden Gelenkarme 120, 130 können dadurch miteinander verbunden werden, dass zwei Teile einer Kopplungsstruktur 150, deren erster Teil am Operationsmikroskop-Kopf 320 des ersten Gelenkarms 120 angeordnet ist und deren zweiter Teil am Laser-Applikator-Kopf 220 des zweiten Gelenkarms 130 angeordnet ist, miteinander in Verbindung gebracht werden. Damit das ophthalmologische Untersuchungs- und Therapiesystem 100 in jeder Position der Gelenkarme 120, 130 seine Stabilität behält und diese nicht etwa wegkippen, weisen die Gelenkarme 120, 130 Gewichtsausgleichstrukturen 145 auf.

Durch diese Gelenkarme hindurch wird auch ein Untersuchungsstrahl einer Vorrichtung für die optische Kohärenztomographie (OCT) geleitet. Dies ist in beiden Gelenkarmen 120, 130 mittels einer Lichtleitfaser möglich. Im Gelenkarm 130, durch den die gepulste Laserstrahlung der Therapielaservorrichtung zum Laser-Applikator-Kopf 220 geleitet wird, ist dies alternativ möglich unter Nutzung der Strahlführungsvorrichtung mit ihrer Strahlfokussieroptik, die von der gepulsten Laserstrahlung genutzt wird. In diesem Fall wird der Untersuchungsstrahl frei durch den Gelenkarm 130 hindurchgeleitet.

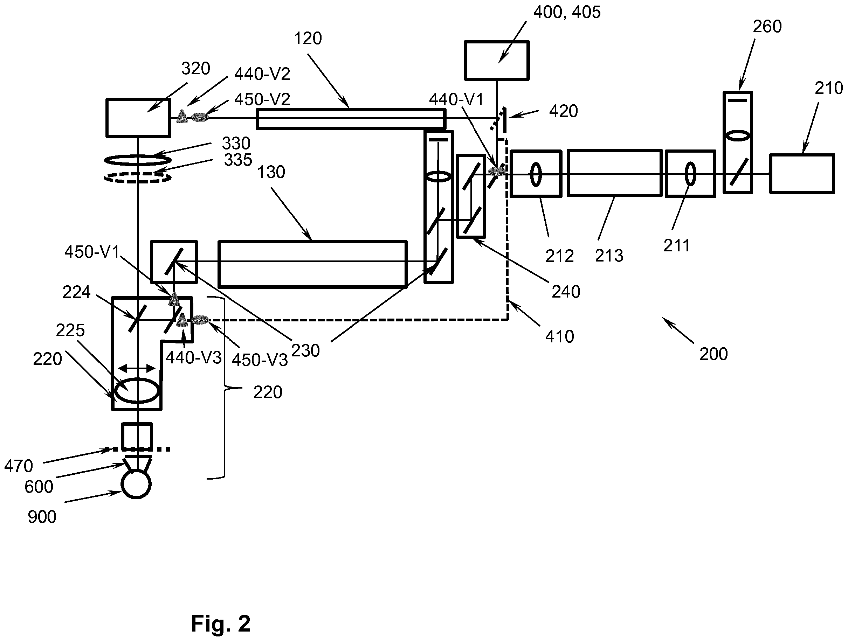

Die Fig. 2 zeigt ein optisches System mit Vorrichtungen zur Erzeugung eines Untersuchungsstrahls bzw. eines Therapielaserstrahls und Strahlführungsvorrichtungen, wie es in einem ophthalmologischen Untersuchungssystem gemäß Fig. 1 eingesetzt werden kann. Ein OCT-Modul 400, hier ein Swept Source (SS) OCT-Modul, das eine OCT-Strahlungsquelle 405 zur Erzeugung eines Untersuchungsstrahls, einen Referenzstrahlengang, ein Interferometer, einen Detektor und ein oder mehrere Schaltstellen 420 enthält, wird durch ein Steuergerät 500 - nicht gezeigt in Fig. 2 - gesteuert. Des Weiteren enthält das System für die Erzeugung des Therapielaserstrahls eine Femtosekunden(fs)-Laserquelle 210. Ein von dieser fs-Laserquelle 210 ausgehender Therapielaserstrahl durchläuft ein erstes optisches System, im Wesentlichen zusammengesetzt aus einem Divergenz-variierendem System 211, einer Relay-Optik 213 sowie einem fokusverstellendem Linsensystem 212. Im Anschluss daran wird der Therapielaserstrahl durch ein x-y-Scansystem 240 in seinem im Target - also ein Auge 900 eines Patienten - beweglichen Fokuspunkt lateral abgelenkt. Durch verschiedene Spiegel 230, 224 wird der Therapielaserstrahl durch den Gelenkarm 130 und schließlich auf eine bewegliche Optik, insbesondere auf eine lateral bewegliche Objektivlinse 225, die in einem Laser-Applikator-Kopf 220 angeordnet ist, geführt. Dort tritt er über ein Patienteninterface 600, das dazu dient, eine Verbindung zwischen dem Untersuchungs- und Therapiesystem 100 mit dem Auge 900 eines Patienten zu schaffen, in das Auge 900 ein.

Das ophthalmologische Untersuchungs- und Therapiesystem 100 enthält weitere Untersuchungsvorrichtungen neben dem OCT-Modul 400: Ein Operationsmikroskop-Kopf 320 befindet sich am Ende eines nur schematisch angedeuteten ersten beweglichen Gelenkarms 120. Eine Objektivlinse 330 und ggf. eine weitere Linse 335 zur Anpassung der Optik des Operationsmikroskop-Kopfes 320, wenn sich im weiteren Strahlengang die Therapielaseroptik befindet, bilden den strahlungstechnischen Ausgang des Operationsmikroskop-Kopfes 320. Auch enthält das Untersuchungs- und Therapiesystem 100 einen konfokalen Detektor 260.

In der Fig. 2 sind dabei verschiedene Varianten der Integration von einem ophthalmologischen Untersuchungssystem 100, das nach den Prinzipien der optischen Kohärenztomographie arbeitet, und einer Lasertherapievorrichtung skizziert, die alternativ aber auch gleichzeitig eingesetzt werden können. Bei gleichzeitigem Einsatz muss selbstverständlich eine Umschaltung zwischen den verschiedenen Varianten erfolgen. Alle Varianten des Untersuchungssystems 100, das nach den Prinzipien der optischen Kohärenztomographie arbeitet, durchlaufen in irgend einer Weise bewegliche Gelenkarme 120, 130 sowie eine bewegliche Optik, insbesondere eine lateral verschiebbare Objektivlinse 225, wodurch sich die optische Weglänge und/oder die Polarisation im Verlaufe der Untersuchung verändern kann.

Die Schaltstellen 420 leiten die von der OCT-Strahlungsquelle 405 abgegebene Strahlung und die vom Auge 900 zurückkommende Strahlung der OCT-Strahlungsquelle 405 in einem ersten Zustand nur über den zweiten Gelenkarm 130 und den Applikator-Kopf 220, und in einem zweiten Zustand nur über den ersten Gelenkarm 120 und den Mikroskop-Kopf 320. Dies ermöglicht beispielsweise die Nutzung des OCT-Moduls zusammen mit dem Operationsmikroskop-Kopf 320 für den Operationsteil des Einsetzens der Intraokularlinse (IOL), bei dem der Applikator-Kopf nicht gebraucht wird und vom Operationsmikroskop-Kopf 320 entkoppelt in einer Parkposition verweilt, und stellt anderseits sicher, dass der Beleuchtungs- und Detektionsstrahlengang des OCT-Moduls 400 für das Setzen der Schnitte mittels des Fokus des fs-Therapielaserstrahls dem Strahlengang des fs-Therapielaserstrahls entspricht, wodurch Ausrichtungsfehler vermieden werden können. Durch die Schaltstelle bzw. Schaltstellen 420 ist dies möglich, ohne dass ein weiteres OCT-Modul integriert werden muss.

In den verschiedenen Varianten des Aufbaus wird dasselbe OCT-Modul 400, das eine Kurzkohärenz-Lichtquelle als OCT-Strahlungsquelle 405 und ein Interferometer enthält, verwendet, wobei der Untersuchungsstrahl, der in der OCT-Strahlungsquelle 405 erzeugt wird, wahlweise in einen Operationsmikroskop-Kopf 320 oder in den Laser-Applikator-Kopf 220 eingekoppelt wird. Entsprechend wird das vom Auge 900 über denselben Weg rückreflektierte Licht der OCT-Strahlungsquelle 405 in einem Interferometer 402 mit dem Referenzstrahl des Referenzarms überlagert und mit einem Detektor detektiert. Das Interferometer mit dem Detektor, das im OCT-Modul 400 enthalten ist, muss dabei nicht in örtlicher Nähe zur OCT-Strahlungsquelle angeordnet sein. Vielmehr ist es günstig, eine Aufteilung des Strahls aus der OCT-Strahlungsquelle 405 mittels eines Strahlteilers in einen Referenzstrahl in einem "Referenzarm" und in einen Untersuchungsstrahl in einem "Signalarm", über den dieser zum Target geführt wird, erst relativ nahe am Target vorzunehmen, wenn sich sonst die optische Weglänge und/oder die Polarisation im "Signalarm" im Vergleich zum "Referenzarm" durch entsprechende Bewegung der eingesetzten Komponenten ändern könnte, und somit ein Offset erzeugt wird, das eine Auswertung der Messung verfälscht.

In einer ersten Variante werden der Therapielaserstrahl der fs-Laserquelle 210 und der Untersuchungsstrahl der OCT- Kurzkohärenz-Strahlungsquelle 405 über den gleichen, Spiegel enthaltenden, zweiten Gelenkarm 130 dem Laser-Applikator-Kopf 220 und über diesen dem Target im Auge 900 zugeführt, das mittels einer Patienten-Schnittstelle 600 mit dem ophthalmologischen Untersuchungssystem 100 verbunden ist. Nach Zusammenführung der Strahlung beider Strahlungsquellen werden dabei beide über den gleichen x/y-Spiegel-Scanner 240 lateral abgelenkt.

Die Änderung der Polarisation wird mittels einer Referenzanordnung gemessen, die eine Einheit 450-V1 aus Strahlteiler im Strahlengang des Untersuchungsstrahls sowie Photodiode hinter einem Polarisator enthält: Der Strahlteiler lenkt dabei einen Teil der Strahlung auf die Photodiode. Diese Einheit 450-V1 befindet sind im Strahlengang hinter einem Kollimator 440-V1, der den Untersuchungsstrahl vor seinem Eintritt in den im Gelenkarm 130 verlaufenden Teil der Strahlführungsvorrichtung kollimiert.

Diese Lösung hat den Vorteil, dass nur eine einzige Strahlführungsvorrichtung 230, hier in Form einer unter Zuhilfenahme von Spiegeln gebildeten Zuleitungsoptik, für den fs-Therapielaserstrahl und den Untersuchungsstrahl der OCT-Strahlungsquelle 405 zum Laser-Applikator-Kopf 220 notwendig ist.

Alternativ zum Spiegel enthaltenden zweiten Gelenkarm 130 kann in einer anderen Variante als Strahlführungsvorrichtung 230 für die Zuführung des Untersuchungsstrahls der OCT-Kurzkohärenz-Strahlungsquelle 405 eine photonische Kristallfaser, also eine Lichtleitfaser 410, verwendet werden, die entweder über eine den ersten Gelenkarm 120 in den Operationsmikroskop-Kopf 320 oder über den zweiten Gelenkarm 130 in den Laser-Applikator-Kopf 220 verläuft. Auch wenn der Untersuchungsstrahl über den zweiten Gelenkarm 130 in den Laser-Applikator-Kopf geleitet werden soll, muss die Strahlführung also nicht unbedingt frei über den x/y-Spiegel-Scanner 240 verlaufen.

Im Applikator-Kopf 220 werden schließlich der fs-Therapielaserstrahl und der Untersuchungsstrahl der OCT-Strahlungsquelle zusammengeführt und über ein lateral bewegliches Objektiv 225 in das Auge 900 geführt. Im Falle der Nutzung einer Lichtleitfaser 410 wird zur Bestimmung der Änderung der Polarisation eine Einheit aus Strahlteiler und Photodiode 450-V2, 450-V3 direkt hinter einem Kollimator 440-V2, 440-V3 am Ende des jeweiligen Gelenkarms 120, 130 und vor dem Austritt aus dem Operationsmikroskop-Kopf 320 bzw. dem Laser-Applikator-Kopf 220 angeordnet.

In allen Varianten wird eine Änderung der optischen Weglänge jedoch mittels einer Referenzmessung bestimmt, indem eine bekannte Fläche oder Struktur aus dem Gesamtsystem als Referenzfläche 470 bzw. Referenzstruktur genutzt wird. Diese ist im Strahlengang nahe der Lage des Auges 900 in einer Position hinter dem Kollimator 440-V1, 440-V2, 440-V3 angeordnet, in der eine einfache Abbildung möglich ist, auf jeden Fall aber hinter dem Bereich, dessen Bewegung zur Änderung der optische Weglänge führt. Wenn die korrekte Lage der Referenzfläche 470 bzw. der Referenzstruktur bekannt ist, kann das OCT-Bild so verschoben werden bzw. die Bilddaten der Abbildung so korrigiert werden, dass die Referenzfläche 470 bzw. die Referenzstruktur an der erwarteten Stelle liegt.

Der Laser-Applikator-Kopf 220 enthält ein lateral scannendes, bewegliches Objektiv 225. In einer Ausführungsvariante ist dabei die Kohärenzlänge der OCT-Strahlungsquelle 405 in Luft größer als 45mm, besonders bevorzugt größer als 60 mm.

Wird mittels Korrekturelementen eine Änderung der optischen Weglänge oder eine Änderung einer Polarisation korrigiert, so werden Korrekturelemente für die Polarisation wie Paddles zwischen OCT-Strahlungsquelle 405 und Analysator bevorzugt in einer Lichtleitfaser 410 eingesetzt, während eine Phasenverzögerungsplatte im freien Strahlengang angeordnet wird. Auch die Korrekturelemente zur Korrektur der Änderung der optischen Weglänge, wie z.B. variierbare Zusatzfaser oder Delay Line, sind prinzipiell im gesamten Bereich einsetzbar. (Die Korrekturelemente werden in Fig. 2 nicht gezeigt.)

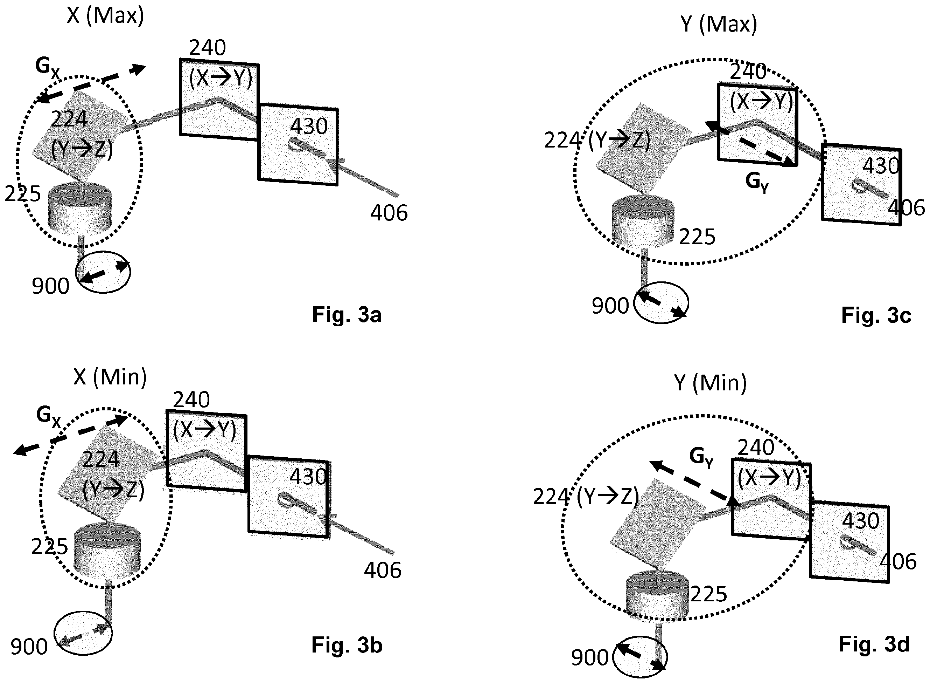

Die Fig. 3a bis 3d zeigen den Einfluss einer beweglichen Objektivlinse 225 auf die optische Weglänge eines Untersuchungsstrahls in einer Strahlführungsvorrichtung eines ophthalmologischen Untersuchungssystems 100. Das Objektiv, bzw. insbesondere die Objektivlinse 225 als Hauptlinse des Objektivs, ist lateral in x- und y-Richtung beweglich. Dabei können vorgeschaltete Umlenkspiegel 224, 240 verwendet werden, deren Abstand zueinander verstellt wird und die sich dabei synchron zur Objektivlinse 225 bewegen. Dies ist in den Fig. 3a bis 3d schematisch dargestellt, die rein exemplarisch auch die Verwendung des Ringspiegels (bzw. Lochspiegels) 430 zeigen. Dieser Ringspiegel 430 ist eine Möglichkeit, den Untersuchungsstrahl 406 mit einem fs-Therapielaserstrahl zu überlagern.

Dabei zeigen die Fig. 3a und 3b die beiden Endpunkte der Verschiebung des Untersuchungsstrahls durch das bewegliche Objektiv 225, durch die eine Änderung der optischen Weglänge OPD des Untersuchungsstrahls bewirkt wird, in x-Richtung. Die Fig. 3c und 3d hingegen zeigen die beiden Endpunkte der Verschiebung in y-Richtung. Dabei ist jeweils auch die Änderung der Position des Untersuchungsstrahls in einem Bildfeld eines Auges 900 gezeigt.

Die Fig. 3a bis 3d zeigen dabei, dass der Linsenkörper des Objektivs 225 fest mit dem Spiegel 224 gekoppelt ist, sich also mit diesem mitbewegt. Dies hat hinsichtlich der Abbildungsqualität Vorteile, da der Linsenkörper des Objektivs 225 immer korrekt zu seiner optischen Achse durchstrahlt wird. Die dreidimensionale Darstellung der Fig. 3a bis 3d lässt die Verstellwirkung der Spiegel 240 und 224 unter Mitführung der jeweils nachgeordneten Elemente (Linsenkörper des Objektivs 225 zusammen mit dem Spiegel 224 bzw. Spiegel 224 und Linsenkörper des Objektivs 225 bei Bewegung des Spiegels 240) gut erkennen.

Um den Einfluss der Bewegung des Objektives 225 auf das OCT-Signal zu kompensieren, werden bevorzugt bei der Berechnung der A-Scans aus den OCT-Signalen die Unterschiede der optischen Weglängen - von bis zu 25 mm, in der Regel jedoch von bis zu 13 mm bei unterschiedlichen Objektiv-Positionen - mit berücksichtigt.

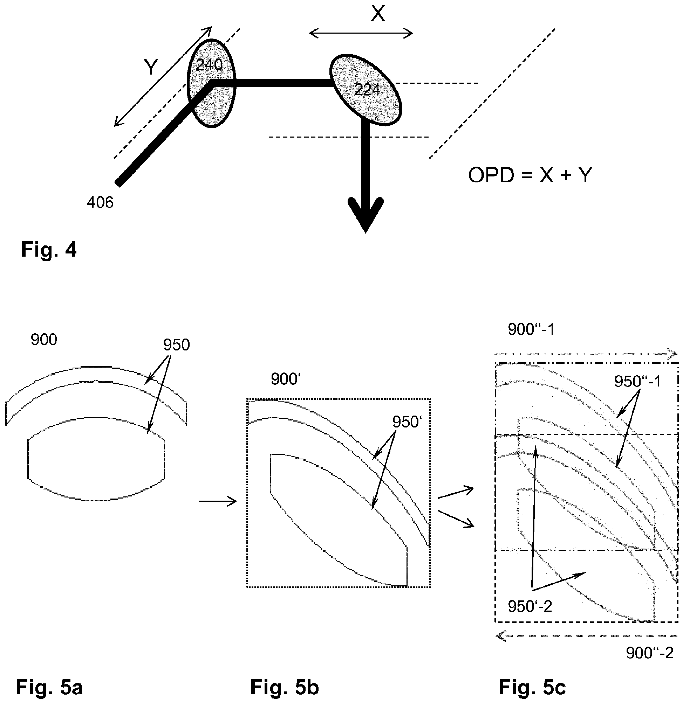

Die Fig. 4 zeigt schematisch die Änderung der optischen Weglänge eines Untersuchungsstrahls, insbesondere eines OCT-Untersuchungsstrahls 406, bei Einsatz einer beweglichen Objektivlinse. Die Änderung der optischen Weglänge OPD setzt sich dabei zusammen aus einem Anteil in x-Richtung und einem Anteil in y-Richtung.

Die Fig. 5a zeigt eine schematisierte Abbildung der Strukturen 950 eines Targets 900, hier einer Cornea und einer Linse eines Auges zunächst so, wie es bei statischen Aufnahmen zu sehen ist - also ohne Bewegungen der Gelenkarme 120, 130 oder einzelner optischer Komponenten wie der Objektivlinse 225, und damit ohne Änderung der optischen Weglänge und/oder der Polarisation während der Untersuchung mittels optischer Kohärenztomographie (OCT). Diese Abbildung ist also die nicht modifizierte Abbildung, d.h. an dieser Stelle die unverzerrte und in der richtigen Position befindliche Abbildung. Sie zeigt die reale Geometrie.

Die Fig. 5b zeigt eine schematisierte (modifizierte) Abbildung der Strukturen 950' des Targets 900' in einem beweglichen Untersuchungssystem 100. Eine Scherung der Abbildung in z-Richtung tritt auf durch den Einfluss der Änderung der optischen Weglänge OPD - dem "Effekt 0. Ordnung" -, wie sie beispielsweise bei einer Bewegung der Objektivlinse 225 während einer Untersuchung mittels optischer Kohärenztomographie erfolgt.

Schließlich zeigt die Fig. 5c eine schematische (modifizierte) Abbildung der Struktur 950"-1 bzw. 950"-2 des Targets 900"-1 bzw. 900"-2, die neben der auftretenden Scherung durch den Einfluss der Änderung der optischen Weglänge OPD noch eine Verschiebung der Abbildung in z-Richtung, also entlang der optischen Achse, aufweist. Diese Verschiebung der Abbildung entsteht durch den Einfluss der Geschwindigkeit der Änderung der optischen Weglänge dOPD/dt - dem "Effekt 1. Ordnung" - bei Einsatz eines OCT-Linienscans als B-Scan in Aneinanderreihung vieler A-Scans. Die Richtung der Verschiebung ist abhängig von der Richtung der Geschwindigkeit der Änderung der optischen Weglänge.

Wie oben schon erläutert, wird bevorzugt ein SweptSource OCT in Kombination mit einer bewegten Optik, insbesondere einer transversal beweglichen Objektivlinse 225 genutzt. Während die einzelnen OCT-Scans aufgenommen werden, bewegt sich diese Optik und es ändert sich die optische Weglänge OPD zum Target 900 (auch während eines A-Scans). Dies führt zum einen zu einer Scherung der Abbildung der Struktur 950 des Targets 900, also des OCT-Bildes, die direkt mit der Änderung der optischen Weglänge OPD korreliert. Diese Änderung kann direkt in die Abbildung eingerechnet werden und die Abbildung damit entzerrt werden.

Durch die Bewegung ergibt sich weiterhin auch eine nichtintuitive Verschiebung der Abbildung in Abhängigkeit von der aktuellen Geschwindigkeit der bewegten Optik, präziser der aktuellen Geschwindigkeit der Änderung der optischen Weglänge, inklusive dessen Vorzeichen. Hierzu wird zu jedem A-Scan die Geschwindigkeit der optischen Weglängenänderung berechnet und über diese und die Sweep-Geschwindigkeit der Strahlungsquelle des SweptSource OCT die jeweilige Zusatzkorrektur berechnet. Für Linienscans mit konstanter Geschwindigkeit ist der Offset konstant, für Kreis-Scans hingegen ortsabhängig, wie unten erläutert.

Auf diese Weise ist es möglich, einen korrekten B-Scan zu ermitteln, obwohl sich während der einzelnen A-Scans die optische Weglänge aktiv ändert.

Konkret bedingt also die Anwendung des SweptSource OCT-Prinzips in einem beweglichen, und folglich nichtstatischen Untersuchungssystem die Korrektur mehrerer Einflüsse, um aus dem Rohsignal korrekte OCT-Bilder zu erzeugen:

1. Die Scherung des OCT-Bildes (auch "Verzeichnen" bzw. "Shearing" genannt) zwischen den verschiedenen A-Scans infolge der Veränderung der optischen Weglänge OPD im Signalarm, also dem aktiven Arm des Interferometers, die letztlich zu einer Verzerrung der Abbildung führt.

2. Auf Grund der gleichzeitigen Änderung der OCT-Frequenz k mit einer Rate $v_K$ = dk/dt und der optischen Weglänge OPD mit einer Geschwindigkeit $v_{OPD}$ = dOPD/dt durch das Scannen in x- und y-Richtung ergeben sich weitere Korrekturterme, die zur Korrektur der "Verschiebung" bzw. Korrektur des OCT-Signals eingerechnet werden müssen.

Nur wenn beide Korrekturen angewendet werden, kommt es zu keiner relevanten Abweichung zwischen der realen Geometrie des gescannten Objekts und den OCT-Bildern bzw. den Bilddaten der OCT-Abbildung. Nur wenn sehr langsam gescannt wird, ist der zweite Term, also der "Effekt 1. Ordnung", vernachlässigbar.

Konkret ergibt sich das Detektorsignal D, d.h. die Intensität der Interferenz von Untersuchungsstrahl und Referenzstrahl, bzw. einer Untersuchungswelle und einer Referenzwelle, zu:

$$D= I_0 \sin(k*OPD)$$

für einen einzelnen Rückstreureflex ($\delta$-Reflex) der Rückstreuintensität $I = I_0 * \delta(OPD)$; bzw.

$$D = \int I(OPD) * \sin(k * OPD)\, dOPD$$

für ein komplexes / mehrschichtiges Streuobjekt,

mit k: Wellenvektor der Messstrahlung $k = 2\pi/\lambda$; (z.B. $2\pi/1060nm = 5.93E6m^{-1}$, $2\pi/1020nm = 6.16E6m^{-1}$)
OPD: Änderung der optischen Weglänge zwischen Signalarm und Referenzarm, auch optische Pfaddifferenz genannt.

Die Fourier-Transformation von D ergibt die gesuchte Darstellung I(OPD) der Tiefenschichtung des Streuobjekts.

Im SweptSource-Verfahren wird k linear durchgestimmt, also:

$$k = k_M + v_k * t$$

mit

| | | |
|---|---|---|
| $k_M$: | mittlerer Wellenvektor zum Startzeitpunkt des Durchstimmens (t=0), (z.B. 6.04E6m$^{-1}$) | |
| $V_k$: | Durchstimm-Geschwindigkeit (Sweep-Geschwindigkeit) dk/dt, (z.B. 0.233E6m$^{-1}$ * 2000Hz = 0.466E9m$^{-1}$s$^{-1}$) | |
| t: | Zeit (t = -T/2..T/2), | |
| T: | Durchstimm-Dauer ("Sweep-Dauer"), | |
| $\Delta k = v_k * T$: | Durchstimm-Bereich ("Sweep-Bereich") | |

Der Durchstimm-Bereich $\Delta k$ ist dabei OPD-auflösungsbestimmend, was unten weiter erläutert wird.

Damit ist $D = \sin((k_M + v_k * t) * OPD)$ bzw. $D = \sin(k_M * OPD + v_k * OPD * t)$

D beinhaltet neben einem konstanten Phasenterm ein zeitlich veränderliches Signal; ein $\delta$-Reflex bei der optischen Weglänge erscheint bei einer Frequenz $\omega$

$$\omega = v_k * OPD \quad (z.B.\ v_K = 466kHz/mm).$$

Aus der Rückstreuintensität $D(\omega)$ wird das gesuchte Signal I(OPD) rekonstruiert mit:

$$I(OPD) = D(\omega / v_k).$$

Bei einer Änderung der optischen Weglänge zwischen verschiedenen A-Scans durch das Scansystem ergibt sich eine Verschiebung im Frequenzraum zwischen den A-Scans, die nach Rücktransformation in der OCT Signalverarbeitung naturgemäß zu einer Verschiebung des registrierten Signals führt. Dies ist in der Fig. 5b im Vergleich zur Fig. 5a (ohne eine solche Änderung der optischen Weglänge) für die Darstellung eines Querschnitts von Strukturen des Auges, hier der Linse und der Cornea, anschaulich dargestellt.

In der Fig. 5b ist ein OCT-Bild zu sehen, bei dem während der B-Scans die zurückgelegte optische Weglänge des Untersuchungsstrahls im Signalarm von A-Scan zu A-Scan verändert (in diesem Falle verlängert) wurde, ohne dass sich das zu messende Target, also das Auge mit seinen Strukturen verändert hat.

Im realen System ändern sich sowohl die Laserfrequenz bzw. der Wellenvektor k als auch die optische Weglänge simultan. Ändert sich während des Durchstimmens des Untersuchungsstrahls die optische Weglänge, die der Untersuchungsstrahl bis zum Target, also dem Rückstreuobjekt, zu durchlaufen hat, linear mit der Zeit, d.h.

$$OPD = OPD_M + v_{OPD} * t,$$

wird das Detektorsignal zu:

$$D = \sin((k_M + v_k * t) * (OPD_M + v_{OPD} * t))$$

bzw

$$D = \sin(k_M * OPD_M + v_k * OPD_M * t + v_{OPD} * k_M * t + v_k * v_{OPD} * t^2)$$

Die Fourieranalyse ergibt dann ein Signal bei:

$$\omega = v_k * OPD_M + v_{OPD} * k_M$$

und erscheint wie ein Signal bei:

$$OPD = \omega/v_k = OPD_M + v_{OPD}*k_M/v_k$$

und weist somit eine Verschiebung von $v_{OPD}*k_M/v_k$ zur realen Position des Targets, also des Rückstreuobjektes auf.

Um hier bei den Beispieldaten zu bleiben: Die Verschiebung beträgt 13ms* $v_{OPD}$; also 13μm bei einer Geschwindigkeit der Änderung der optischen Weglänge $v_{OPD}$ von 1mm s$^{-1}$, bzw. 130μm bei einer Geschwindigkeit der optischen Weglänge $v_{OPD}$ von 10mms$^{-1}$. Das ist bei einer geforderten Messgenauigkeit des OCT von wenigen 10μm nicht vernachlässigbar.

Zur Veranschaulichung ist in der Fig. 5c zu Fig. 5b der Vergleich zwischen statisch abgetasteter Geometrie und dem Abscannen mit Änderung der optischen Weglänge mit einer Geschwindigkeit $v_{OPD}$ dargestellt. Die in Fig. 5b dargestellte Scherung der Abbildung infolge der Änderung der optischen Weglänge wird zusätzlich je nach Richtung der Geschwindigkeit $v_{OPD}$ mit- oder entgegen der Richtung der Durchstimm-Geschwindigkeit $v_k$ verschoben.

Um dies zu kompensieren, wird wie folgt vorgegangen: Bei bekannter Änderung der optischen Weglänge OPD zum Target, also zum Rückstreuobjekt, pro Zeit infolge der x-y Bewegung des Scansystems kann das Signal I(OPD) einfach um -$v_{OPD}*k_M/v_k$ in der optischen Weglänge verschoben werden, um das reale Streusignal zu erhalten. Dabei ist zu beachten, dass die Geschwindigkeitsaddition nichteuklidisch erfolgt, also $v_{OPD} = v_x + v_y$, da sich die optische Weglänge OPD mit der Scanbewegung in x additiv zur Scanbewegung in y ergibt, und somit z.B. also auch $v_{OPD}$=0 sein kann, wenn $v_x = -v_y$ obwohl sich der Untersuchungsstrahl über das Target lateral bewegt.

Diese Verschiebung kann mit bekannten Sweep-Parametern $k_M$, $v_k$ einfach erfolgen. Experimentell kann die Verschiebung mit bekannter Scangeschwindigkeit $v_{OPD}$ in eine Richtung und -$v_{OPD}$ in entgegengesetzter Richtung zu einer

Verschiebung 2*$v_{OPD}*\Delta k /v_k$ zusammengezogen werden, und daraus der geschwindigkeitsproportional anzuwendende Korrekturfaktor $\Delta k /v_k$ experimentell ermittelt werden.

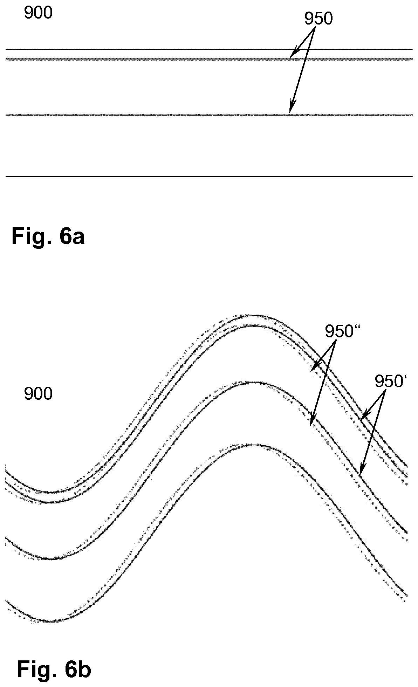

Das Beispiel der Fig. 6a und 6b zeigt ein schematisiertes Bild der Strukturen 950 eines Auges 900 - hier einer Cornea und einer Linse - nicht modifiziert, d.h. hier unverzerrt und in der richtigen Position (Fig. 6a); mit Scherung durch den Einfluss der Änderung der optischen Weglänge in den durchgehenden Linien der Fig. 6b (Strukturen 950'); und zusätzlich verschoben durch den Einfluss der Geschwindigkeit der Änderung der optischen Weglänge in den gepunkteten Linien der Fig. 6b (Strukturen 950") für ein "Chirped" OCT mit Kreisscan.

Dabei ist z. B.: x=Rsin($\Omega$t) und y=Rcos($\Omega$t).

Die Änderung, die die optische Weglänge erfährt, ist dann:

$$\Delta OPD = x + y$$

Die Geschwindigkeit der Änderung der optischen Weglänge ist beschrieben mit

$$v_{OPD}= R\Omega \sqrt{2 \sin(\frac{\pi}{4} - \Omega t)}$$

Das Bild muss demnach mit einer sinusförmigen Korrektur versehen werden, um die Scherung und die Verschiebung, die durch die Änderung der optischen Weglänge und die Geschwindigkeit dieser Änderung entstanden ist, auszugleichen.

Bei einem kreisförmigen Scan wie er beispielsweise für eine Kapsulotomie nötig ist, ändert sich die optische Weglänge nicht linear. Aus der nicht linearen Änderung ergibt sich auch eine variierende Geschwindigkeit der Änderung der optischen Weglänge, die während des Kreisscans zudem das Vorzeichen ändert. Dies ist in den Figuren 6a und 6b verdeutlicht. In der Fig. 6a sind die Strukturen 950, also die Grenzflächen der Kornea und die der Augenlinse so dargestellt, wie es bei einer perfekt zentrierten Cornea und Linse zu erwarten wäre, wenn in einem statischen Untersuchungssystem gemessen würde. In der Fig. 6b erkennt man in den Strukturen 950' mit durchgehenden Linien die Abbildung, die sich nur aufgrund der Änderung der optischen Weglänge bei einem Kreisscan ohne Korrektur ergeben würde. Die Strukturen 950", die mit gepunkteten Linien dargestellt sind, enthalten den zusätzlichen Effekt der Verschiebung aufgrund der veränderlichen Geschwindigkeit der Änderung der optischen Weglänge während eines Kreis-cans.

In der Fig. 6b ist deutlich zu erkennen, dass die Verschiebung und die Scherung in beiden Richtungen möglich sind. An den Stellen, an denen die optische Weglänge konstant ist, ist die resultierende Scherung und Verschiebung erwartungsgemäß Null. Es wird deutlich, dass im Falle eines Kreisscans eine variable Korrektur der Scherung und der Verschiebung über den Scan nötig ist.

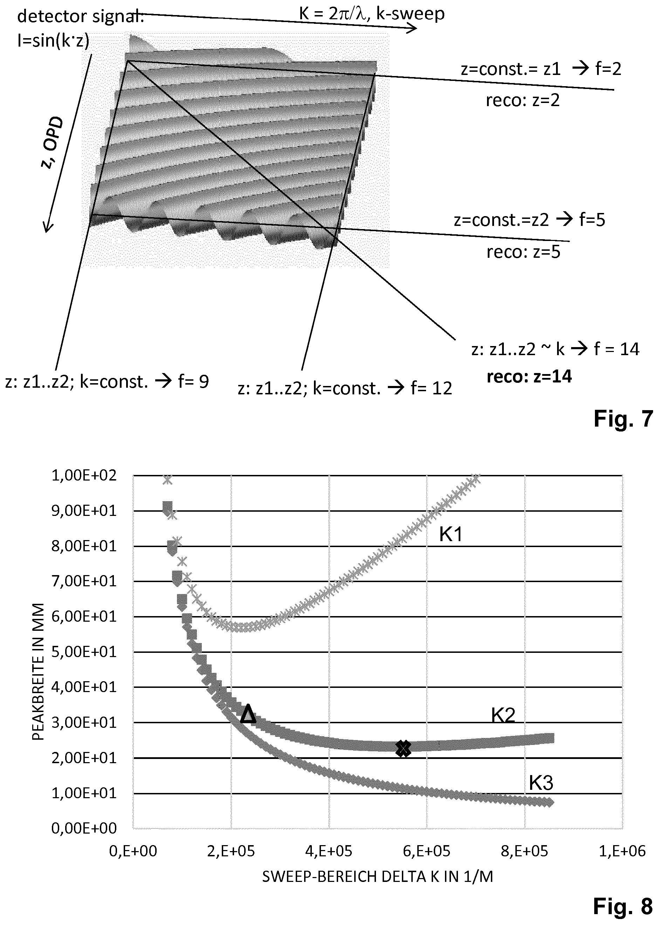

Die Fig. 7 verdeutlicht den Einfluss der Geschwindigkeit in z-Richtung, also entlang der optischen Achse, auf ein Swept-Source OCT-Signal; Die Scherung in z-Richtung ist proportional der Änderung der optischen Weglänge während eines A-Scans, die Verschiebung in z-Richtung ist proportional der Geschwindigkeit der Änderung der optischen Weglänge.

In einem Swept Source OCT-Verfahren wird während der Untersuchung immer wieder ein Frequenzbereich (bzw. Wellenlängenbereich) durchlaufen.

Die Abhängigkeit der Auflösung der optischen Weglänge, insbesondere des Auflösungsoptimums, vom Durchstimmbereich ist in der Fig. 8 dargestellt. Im Vergleich zu einer statischen OCT-Messung weist eine OCT-Messung in einem beweglichen System, in dem sich die optische Weglänge während der Untersuchung ändert, ein Auflösungsoptimum auf: Die Peakbreite verbreitert sich aufgrund der Geschwindigkeitsänderung, weshalb eine Auflösungsverringerung auftritt. Es ist deshalb günstig, einen Sweep-Bereich zu definieren, bei dem ein Auflösungsoptimum der Abbildung erreicht wird.

Zusätzlich variiert das $\omega$ beim Durchstimmen um +/- $v_k$*$v_{OPD}$*T/2, siehe quadratischer t-Term. Das entspricht einem linear veränderlichen $\omega$, was eine Peak-Verbreiterung im Fourier-transformierten Signal und damit eine Auflösungsverringerung der optischen Kohärenztomographie um $d_{OPD}$= $v_{OPD}$*$\Delta k$/$v_k$ = $v_{OPD}$ *T bewirkt. Das entspricht dem intuitiv erwarteten Überstreichen des statischen Signal-Peaks über die Änderung der optischen Weglänge während der Zeit des A-Scans T, also z.B. 0.5$\mu$m bei 1mm/s bzw. 5$\mu$m bei 10mm/s. Die Verringerung der OCT-Auflösung von bis zu 5$\mu$m ist bei einer geforderten Messgenauigkeit des OCT von wenigen 10$\mu$m gerade noch tolerierbar.

Die Fig. 8 zeigt nun die Abhängigkeit der Auflösung der optischen Weglänge vom Sweep-Bereich $\Delta k$. Die Kurve K3 zeigt eine "statische" Kurve - ohne Änderung der optischen Weglänge während des A-Scans. Die Kurve K2 ist eine Kurve mit Änderung der optischen Weglänge während eines A-Scans - mit einem typischen Wert der Änderungsgeschwindigkeit der optischen Weglänge von $v_{OPD}$=10mm/s mit einem Arbeitspunkt bei 2.3E5m$^{-1}$ (siehe großes Dreieck) und einem Minimum (siehe Kreuz). Die Kurve K1 ist eine Kurve mit Änderung der optischen Weglänge während des A-Scans, mit einem hohen Wert der Änderungsgeschwindigkeit der optischen Weglänge von $v_{OPD}$ = 60mm/s. Das Minimum der Peakbreite liegt vor dem 2.3E5m$^{-1}$-Arbeitspunkt, eine Verringerung der Durchstimm-Breite auf 2.2E4m$^{-1}$ ist vorteilhaft.

Während bei statischer OCT-Messung die Auflösung (Peakbreite w eines $\delta$-Reflexes) durch den Durchstimm-Bereich $\Delta k$ bestimmt wird: $w_s$=2$\pi$/$\Delta k$ und bei größeren Durchstimm-Bereichen feiner wird, ist die Verbreiterung aufgrund der Änderung der optischen Weglänge bei größeren Durchstimm-Bereichen größer (unter Annahme einer konstanten

Durchstimm-Geschwindigkeit $v_k$). Deshalb ergibt sich ein Auflösungsoptimum bei $\Delta k = \sqrt{\left(2\pi \frac{v_k}{v_{OPD}}\right)}$ .

Mit den oben angeführten Beispieldaten für $v_k$ und $v_{OPD}$ liegt das Optimum bei $\Delta k$=0.541E6m$^{-1}$, dem doppelten Sweep-Bereich, den die Laser-Quelle bietet. Erst ab Scan-Geschwindigkeiten > 55mm/s und damit $v_{OPD}$ > 55mm/s liegt das Optimum vor den benutzten 0.233E6m$^{-1}$ Durchstimm-Bereich, und eine Einschränkung des Durchstimm-Bereiches würde vorteilhaft werden: Eine solche Einschränkung führt in diesem Falle zu einer bessere Auflösung, einer geringeren Durchstimm-Zeit und damit einer höheren A-Scan Wiederholrate.

Während man also bei einer statischen Messung eine bzgl. Frequenzbereich bzw. k-Range möglichst breite Swept-Source nutzen würde, um eine möglichst hohe Auflösung zu erreichen, kann dies bei einer SweptSoure OCT-Untersuchung, bei der sich die optische Weglänge ändert, weil beispielsweise das Objektiv während der Untersuchung lateral verschoben wird, dazu führen, dass ein zu breiter Durchstimm-Bereich die Auflösung nicht erhöht sondern diese sogar verschlechtert.

Die Durchstimm-Geschwindigkeit sollte immer auf dem höchsten technisch beherrschbaren Wert gehalten werden, um geringe Auflösungseinbußen bei $v_{OPD}$, und eine höhere A-Scan-Wiederholrate zu erreichen. Begrenzend sind die maximale Durchstimm-Rate der Laserquelle und die Grenzfrequenz der Detektoren inklusive der Datenaufnahme.

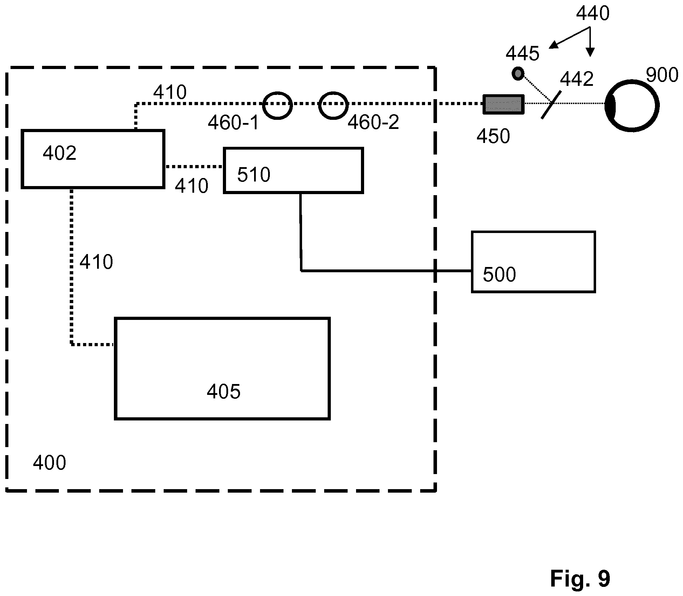

Die Fig. 9 zeigt eine Ausführungsform eines OCT-Moduls eines erfindungsgemäßen ophthalmologischen Untersuchungssystems. Das OCT-Modul enthält eine OCT-Strahlungsquelle 405 als Vorrichtung zur Erzeugung eines Untersuchungsstrahls, ein Interferometer 402 zur Analyse durch Überlagerung des vom Target 900 zurückgesendeten Untersuchungsstrahls mit einem Referenzstrahl, eine Signalverarbeitungseinheit 510 bestehend aus einer Datenerfassungskarte in Kombination mit Photodioden und elektrischen Verstärkern, das die ermittelten Daten digitalisiert, und an die Steuereinheit 500 übergibt. Das Interferometer 402 ist mit der OCT-Strahlungsquelle 405, der Signalverarbeitungseinheit 510 und schließlich dem Weg zum Auge mittels Lichtleitfasern 410 verbunden. Am Austritt der Lichtleitfaser ist ein Kollimator 450 angeordnet. Dieser sendet die Strahlung durch einen Strahlteiler 442 auf das Target 900, also das Auge. Zum anderen wird ein Teil der Strahlung auf die Photodiode 445 reflektiert, die als Referenzanordnung dient. Bei Feststellung einer Änderung der Polarisierung des Untersuchungsstrahls wird diese über zwei bewegliche Paddels 460-1, 460-2 korrigiert, um wieder die ursprüngliche Polarisation zu erreichen.

Die vorstehend genannten und in verschiedenen Ausführungsbeispielen erläuterten Merkmale der Erfindung sind dabei nicht nur in den beispielhaft angegebenen Kombinationen, sondern auch in anderen Kombinationen oder allein einsetzbar.

Eine auf Verfahrensmerkmale bezogene Beschreibung einer Vorrichtung gilt bezüglich dieser Merkmale analog für das entsprechende Verfahren, während Verfahrensmerkmale entsprechend funktionelle Merkmale der beschriebenen Vorrichtung darstellen.

**Patentansprüche**

1. Verfahren zur direkten Korrektur einer Abbildung, die auf einer Untersuchung eines Targets mit einem Untersuchungsstrahl (406) einer Vorrichtung für die optische Kohärenztomographie(OCT) basiert,

   - wobei eine Abbildung einer Struktur (950) des Targets (900) verwendet wird bzw. Bilddaten einer Abbildung der Struktur des Targets (900) verwendet werden, zu deren Erzeugung eine zurückgelegte optische Weglänge von einer Quelle des Untersuchungsstrahls zu der Struktur des Targets (900) und/oder von der Struktur des Targets (900) zu einer Analysevorrichtung ausgewertet wird,
   - wobei sich die optische Weglänge des Untersuchungsstrahls (406) zum und/oder vom Target (900) im Verlaufe der Erzeugung der Abbildung der Struktur (950) des Targets (900) diskret oder kontinuierlich ändert,
   - und die Änderung der optischen Weglänge (OPD) des Untersuchungsstrahls (406) zum und/oder vom Target (900) bekannt ist oder gemessen wird, und
   - die bekannte oder gemessene Änderung zur Einstellung eines verstellbaren Korrekturelements oder aber zur direkten Korrektur der Abbildung bzw. der Bilddaten der Abbildung verwendet wird, derart, dass der Einfluss der Änderung der optischen Weglänge (OPD) des Untersuchungsstrahls (406) zum und/oder vom Target (900) auf die Abbildung ausgeglichen wird
   - wobei der Untersuchungsstrahl (406) frei durch ein in seiner internen Lagebeziehung seiner Komponenten variierbares System geleitet wird, sich während der Lagebeziehungsänderung des Systems die optische Weglänge des Untersuchungsstrahls (406) ändert, und eine dabei entstehende Scherung der Abbildung in Abhängigkeit von der Änderung der optischen Weglänge (OPD) korrigiert wird, und
   - insbesondere eine mit einem "Time-Domain" (TD)-OCT- oder "Swept Source" (SS)-OCT-Verfahren erzeugte Abbildung genutzt wird bzw. Bilddaten einer solchen Abbildung genutzt werden, bei denen eine Verschiebung der Abbildung in Abhängigkeit von der Geschwindigkeit der Änderung der optischen Weglänge (dOPD/dt) direkt korrigiert wird.

2. Verfahren nach Anspruch 1,

   - wobei die Abbildung der Struktur (950) des Targets (900) verwendet wird bzw. Bilddaten der Abbildung der Struktur des Targets (900) verwendet werden, zu deren Erzeugung eine Polarisation des Untersuchungsstrahls (406) ausgewertet wird,
   - wobei sich die Polarisation des Untersuchungsstrahls (406) im Verlaufe der Erzeugung der Abbildung der Struktur (950) des Targets (900) diskret oder kontinuierlich ändert,
   - und die Änderung der Polarisation bekannt ist oder gemessen wird, und
   - die bekannte oder gemessene Änderung zur Einstellung eines verstellbaren Korrekturelements oder aber zur direkten Korrektur der Abbildung bzw. der Bilddaten der Abbildung verwendet wird, derart, dass der Einfluss der Änderung der Polarisation des Untersuchungsstrahls (406) auf die Abbildung ausgeglichen wird.

3. Verfahren nach Anspruch 2, wobei der Untersuchungsstrahl mittels Lichtleitfaser (410) und/oder frei durch ein in seiner internen Lagebeziehung seiner Komponenten variierbares System geleitet und eine während einer Lagebeziehungsänderung des Systems entstehende Änderung der Polarisation des Untersuchungsstrahls durch zwei bewegliche Paddles (460) oder durch eine Phasenverzögerungsplatte korrigiert wird.

4. Verfahren nach einem der Ansprüche 1 bis 3, wobei der Untersuchungsstrahl (406) frei durch ein in seiner internen Lagebeziehung seiner Komponenten variierbares System geleitet wird, und eine während einer Lagebeziehungsänderung des Systems entstehende Änderung der optischen Weglänge (OPD) durch eine variierbare Zusatzfaser oder eine Delay Line korrigiert wird.

5. Ophthalmologisches Untersuchungssystem (100), das eine Vorrichtung für die optische Kohärenztomographie (OCT) umfasst, und

   - eine Vorrichtung zur Erzeugung eines Untersuchungsstrahls (405),
   - eine Analysevorrichtung für die Analyse des von einer Struktur eines Targets zurückgesendeten Untersuchungsstrahls (406) und zur Erzeugung von Bilddaten einer Abbildung der Struktur des Targets

- eine Strahlführungsvorrichtung zur Führung des Untersuchungsstrahls (406) von der Vorrichtung zur Erzeugung (405) des Untersuchungsstrahls zum Target und vom Target zur Analysevorrichtung (402) enthält, wobei eine optische Weglänge des Untersuchungsstrahls (406) in der Strahlführungsvorrichtung diskret oder kontinuierlich veränderlich ist,

- eine Steuervorrichtung (500) zur Steuerung des ophthalmologischen Untersuchungssystems, wobei

- die diskrete oder kontinuierliche Veränderung der optischen Weglänge in Abhängigkeit von der Zeit bekannt oder mittels einer Referenzanordnung messbar ist, und

- die Steuervorrichtung eine Korrektureinheit eingerichtet zur direkten Korrektur der Abbildung oder der Bilddaten der Abbildung in Abhängigkeit von der Änderung der optischen Weglänge (OPD) des Untersuchungsstrahls (406) (406) in der Strahlführungsvorrichtung aufweist, wobei

die Korrektureinheit eingerichtet ist, eine in Abhängigkeit von der Änderung der optischen Weglänge (OPD) auftretende Scherung der optischen Kohärenztomographie-Abbildung direkt zu korrigieren, und dessen Vorrichtung für die optische Kohärenztomographie insbesondere eine Vorrichtung für "Time-Domain" (TD)-OCT oder "Swept Source" (SS)-OCT umfasst, und die Korrektureinheit eingerichtet ist, eine Verschiebung der optischen Kohärenztomographie-Abbildung in Abhängigkeit von der Geschwindigkeit der Änderung der optischen Weglänge (dOPD/dt) direkt zu korrigieren.

6. Ophthalmologisches Untersuchungssystem (100) nach Anspruch 5, ferner umfassend ein verstellbares Korrekturelement zum Ausgleich der Änderung der optischen Weglänge (OPD).

7. Ophthalmologisches Untersuchungssystem (100) nach Anspruch 5 oder 6,

- wobei eine Polarisation des Untersuchungsstrahls (406) in der Strahlführungsvorrichtung diskret oder kontinuierlich veränderlich ist,

- wobei die diskrete oder kontinuierliche Veränderung der Polarisation in Abhängigkeit von der Zeit bekannt oder mittels einer Referenzanordnung messbar ist, und

- das ophthalmologische Untersuchungssystem (100) ein verstellbares Korrekturelement zum Ausgleich der Änderung der Polarisation und/oder die Steuervorrichtung eine Korrektureinheit eingerichtet zur direkten Korrektur der Abbildung oder der Bilddaten der Abbildung in Abhängigkeit von der Änderung der Polarisation des Untersuchungsstrahls (406) in der Strahlführungsvorrichtung aufweist.

8. Ophthalmologisches Untersuchungssystem (100) nach Anspruch 6, wobei das verstellbare Korrekturelement eine Delay Line zum Ausgleich der Änderung der optischen Weglänge (OPD) des Untersuchungsstrahls (406) umfasst und das verstellbare Korrekturelement in der Strahlführungsvorrichtung und/oder in einer alternativen Strahlführungsvorrichtung für einen Referenzstrahl enthalten ist.

9. Ophthalmologisches Untersuchungssystem (100) nach Anspruch 7, wobei das verstellbare Korrekturelement zwei bewegliche Paddles (460) oder eine Phasenverzögerungsplatte zum Ausgleich der Änderung der Polarisation umfasst und das verstellbare Korrekturelement in der Strahlführungsvorrichtung und/oder in einer alternativen Strahlführungsvorrichtung für einen Referenzstrahl enthalten ist.

10. Ophthalmologisches Untersuchungssystem (100) nach einem der Ansprüche 5 bis 7, das weiterhin eine Anzeigevorrichtung zur Sichtbarmachung der Abbildung mit Hilfe der Bilddaten der Abbildung einer Struktur (950) des Targets (900) enthält.

11. Ophthalmologisches Untersuchungssystem (100) nach einem der Ansprüche 5 bis 10, wobei die Strahlführungsvorrichtung ein in seiner internen Lagebeziehung seiner Komponenten variierbares System aufweist und eine Lichtleitfaser (410) enthält.

12. Ophthalmologisches Untersuchungssystem (100) nach einem der Ansprüche 5 bis 11, wobei die Strahlführungsvorrichtung ein in seiner internen Lagebeziehung seiner Komponenten variierbares System aufweist und mindestens ein Teil der Strahlführungsvorrichtung eingerichtet ist, den Untersuchungsstrahl frei durch das in seiner internen Lagebeziehung seiner Komponenten variierbare System zu führen.

13. Ophthalmologisches Untersuchungssystem (100) nach einem der Ansprüche 5 bis 12, dessen Strahlführungsvorrichtung eine bewegliche Optik, insbesondere eine lateral bewegliche Objektivlinse (225) enthält.

14. Ophthalmologisches Untersuchungssystem (100) nach einem der Ansprüche 5 bis 13, dessen Vorrichtung für die optische Kohärenztomographie (OCT) in abgeschlossene, unabhängig voneinander austauschbare und einzeln korrigierbare Teilsysteme mit definierten Übergängen unterteilt ist, bevorzugt in ein erstes Teilsystem, das ein Interferometer (402) enthält und ein zweites Teilsystem, das eine Lichtleitfaser (410) mit einem Kollimator (450) umfasst oder alternativ bevorzugt in ein erstes Teilsystem, das ein Interferometer (402) enthält, ein zweites Teilsystem, das eine lange Lichtleitfaser (410) enthält, die durch ein in seiner internen Lagebeziehung seiner Komponenten variierbares System hindurch verläuft, und in ein drittes Teilsystem, dass eine kurze Lichtleitfaser (410) und einen Kollimator (450) umfasst.

15. Ophthalmologisches Untersuchungssystem (100) nach einem der Ansprüche 5 bis 14, das eine Therapielaservorrichtung (200) umfasst, wobei eine Vorrichtung zur Erzeugung des Untersuchungsstrahls (406) ebenfalls zur Erzeugung des Therapielaserstrahls eingerichtet ist oder eine unabhängige Vorrichtung zur Erzeugung (210) des Therapielaserstrahls neben der Vorrichtung zur Erzeugung (405) des Untersuchungsstrahls enthalten ist.

## Claims

1. Method for directly correcting an image that is based on an examination of a target using an examination beam (406) of an optical coherence tomography (OCT) device,

   - wherein an image of a structure (950) of the target (900) is used or image data of an image of the structure of the target (900) are used, for the creation of which an optical path length travelled from a source of the examination beam to the structure of the target (900) and/or from the structure of the target (900) to an analysis device is evaluated,
   - wherein the optical path length of the examination beam (406) to and/or from the target (900) changes discretely or continuously in the course of the creation of the image of the structure (950) of the target (900),
   - and the change in optical path length (OPD) of the examination beam (406) to and/or from the target (900) is known or is measured, and
   - the known or measured change is used to set an adjustable correction element or else to directly correct the image or the image data of the image, in such a way that the influence of the change in optical path length (OPD) of the examination beam (406) to and/or from the target (900) on the image is compensated for,
   - wherein the examination beam (406) is freely guided through a system that can be varied in terms of its internal positional relationship of its components, the optical path length of the examination beam (406) changes during the change in positional relationship of the system, and a resulting shearing of the image is corrected for on the basis of the change in optical path length (OPD), and
   - in particular an image created using a "Time-Domain" (TD)-OCT or "Swept Source" (SS)-OCT method is used or image data of such an image are used, in which a displacement of the image is directly corrected on the basis of the rate of change of the optical path length (dOPD/dt).

2. Method according to Claim 1,

   - wherein the image of the structure (950) of the target (900) is used or image data of the image of the structure of the target (900) are used, for the creation of which a polarization of the examination beam (406) is evaluated,
   - wherein the polarization of the examination beam (406) changes discretely or continuously in the course of the creation of the image of the structure (950) of the target (900),
   - and the change in polarization is known or is measured, and
   - the known or measured change is used to set an adjustable correction element or else to directly correct the image or the image data of the image, in such a way that the influence of the change in polarization of the examination beam (406) on the image is compensated for.

3. Method according to Claim 2, wherein the examination beam is guided by means of an optical fibre (410) and/or freely through a system that can be varied in terms of its internal positional relationship of its components, and a change in polarization of the examination beam resulting during a change in positional relationship of the system is corrected by two movable paddles (460) or by a phase retardation plate.

4. Method according to any of claims 1 to 3, wherein the examination beam (406) is freely guided through a system that can be varied in terms of its internal positional relationship of its components, and a change in optical path length (OPD) resulting during a change in positional relationship of the system is corrected by a variable additional fibre or a delay line.

5. Ophthalmological examination system (100) comprising an optical coherence tomography (OCT) device, and containing

- a device for creating an examination beam (405),
- an analysis device for analysing the examination beam (406) returned by a structure of a target and for creating image data of an image of the structure of the target,
- a beam guiding device for guiding the examination beam (406) from the device for creating (405) the examination beam to the target and from the target to the analysis device (402), wherein an optical path length of the examination beam (406) in the beam guiding device is discretely or continuously changeable,
- a control device (500) for controlling the ophthalmological examination system, wherein
- the discrete or continuous change in optical path length as a function of time is known or measurable by means of a reference arrangement, and
- the control device comprises a correction unit configured to directly correct the image or the image data of the image on the basis of the change in optical path length (OPD) of the examination beam (406) (406) in the beam guiding device, wherein

the correction unit is configured to directly correct shearing of the optical coherence tomography image that occurs dependent on the change in optical path length (OPD),
and its optical coherence tomography device in particular comprises a device for "Time-Domain" (TD)-OCT or "Swept Source" (SS)-OCT, and the correction unit is configured to directly correct a displacement of the optical coherence tomography image on the basis of the rate of change of the optical path length (dOPD/dt).

6. Ophthalmological examination system (100) according to Claim 5, further comprising an adjustable correction element for compensating for the change in optical path length (OPD).

7. Ophthalmological examination system (100) according to Claim 5 or 6,

- wherein a polarization of the examination beam (406) in the beam guiding device is discretely or continuously changeable,
- wherein the discrete or continuous change in polarization as a function of time is known or measurable by means of a reference arrangement, and
- the ophthalmological examination system (100) comprises an adjustable correction element for compensating for the change in polarization, and/or the control device comprises a correction unit configured for directly correcting the image or the image data of the image on the basis of the change in polarization of the examination beam (406) in the beam guiding device.

8. Ophthalmological examination system (100) according to Claim 6, wherein the adjustable correction element comprises a delay line for compensating for the change in optical path length (OPD) of the examination beam (406), and the adjustable correction element is contained in the beam guiding device and/or in an alternative beam guiding device for a reference beam.

9. Ophthalmological examination system (100) according to Claim 7, wherein the adjustable correction element comprises two movable paddles (460) or a phase retardation plate for compensating for the change in polarization, and the adjustable correction element is contained in the beam guiding device and/or in an alternative beam guiding device for a reference beam.

10. Ophthalmological examination system (100) according to any of Claims 5 to 7, furthermore containing a display device for rendering the image visible with the aid of the image data of the image of a structure (950) of the target (900).

11. Ophthalmological examination system (100) according to any of Claims 5 to 10, wherein the beam guiding device both comprises a system that can be varied in terms of its internal positional relationship of its components and contains an optical fibre (410).

12. Ophthalmological examination system (100) according to any of Claims 5 to 11, wherein the beam guiding device comprises a system that can be varied in terms of its internal positional relationship of its components, and at least a part of the beam guiding device is configured to guide the examination beam freely through the system that can be varied in terms of its internal positional relationship of its components.

13. Ophthalmological examination system (100) according to any of Claims 5 to 12, the beam guiding device of which contains a movable optical unit, in particular a laterally movable objective lens (225).

14. Ophthalmological examination system (100) according to any of Claims 5 to 13, whose optical coherence tomography (OCT) device is subdivided into closed, independently interchangeable and individually correctable subsystems with defined transitions, preferably into a first subsystem containing an interferometer (402) and a second subsystem comprising an optical fibre (410) with a collimator (450) or, in an alternative to that, preferably into a first subsystem containing an interferometer (402), a second subsystem containing a long optical fibre (410), which passes through a system that can be varied in terms of its internal positional relationship of its components, and into a third subsystem comprising a short optical fibre (410) and a collimator (450).

15. Ophthalmological examination system (100) according to any of Claims 5 to 14, comprising a therapy laser device (200), wherein a device for creating the examination beam (406) is also configured to create the therapy laser beam, or an independent device for creating (210) the therapy laser beam is included beside the device for creating (405) the examination beam.

**Revendications**

1. Procédé de correction directe d'une image, qui se base sur l'examen d'une cible avec un faisceau d'examen (406) d'un dispositif de tomographie par cohérence optique (OCT),

- une image d'une structure (950) de la cible (900) étant utilisée ou des données d'image d'une image de la structure de la cible (900) étant utilisées, une longueur de trajet optique parcourue depuis une source du faisceau d'examen jusqu'à la structure de la cible (900) et/ou depuis la structure de la cible (900) jusqu'à un dispositif d'analyse étant évaluée pour la génération de celles-ci,
- la longueur de trajet optique du faisceau d'examen (406) vers et/ou depuis la cible (900) variant de manière discrète ou continue au cours de la génération de l'image de la structure (950) de la cible (900),
- et la variation de la longueur de trajet optique (OPD) du faisceau d'examen (406) vers et/ou depuis la cible (900) étant connue ou mesurée, et
- la variation connue ou mesurée étant utilisée pour régler un élément de correction ajustable ou encore pour corriger directement l'image ou les données d'image de l'image, de telle sorte que l'influence sur l'image de la variation de la longueur de trajet optique (OPD) du faisceau d'examen (406) vers et/ou depuis la cible (900) est compensée
- le faisceau d'examen (406) étant guidé librement à travers un système dont la relation de position interne de ses composants peut être modifiée, la longueur de trajet optique du faisceau d'examen (406) variant pendant la modification de la relation de position du système, et un cisaillement ainsi produit de l'image étant corrigé en fonction de la variation de la longueur de trajet optique (OPD), et
- une image produite par un procédé d'OCT « en domaine temporel » (TD) ou d'OCT à « source balayée » (SS) étant notamment utilisée ou des données d'image d'une telle image étant notamment utilisées, avec lesquelles un décalage de l'image en fonction de la vitesse de variation de la longueur de trajet optique (dOPD/dt) est directement corrigé.

2. Procédé selon la revendication 1,

- l'image de la structure (950) de la cible (900) étant utilisée ou des données d'image de l'image de la structure de la cible (900) étant utilisées, pour la production desquelles une polarisation du faisceau d'examen (406) est évaluée,
- la polarisation du faisceau d'examen (406) variant de manière discrète ou continue au cours de la génération de l'image de la structure (950) de la cible (900),
- et la variation de la polarisation étant connue ou mesurée, et
- la variation connue ou mesurée étant utilisée pour régler un élément de correction ajustable ou encore pour corriger directement l'image ou les données d'image de l'image, de telle sorte que l'influence sur l'image de la variation de la polarisation du faisceau d'examen (406) est compensée.

3. Procédé selon la revendication 2, le faisceau d'examen étant guidé au moyen d'une fibre optique (410) et/ou librement à travers un système dont la relation de position interne de ses composants peut être modifiée, et une variation de la polarisation du faisceau d'examen qui se produit pendant une modification de la relation de position du système étant corrigée par deux palettes mobiles (460) ou par une plaque à retard de phase.

4. Procédé selon l'une des revendications 1 à 3, le faisceau d'examen (406) étant guidé librement à travers un système dont la relation de position interne de ses composants peut être modifiée, et une modification de la longueur de trajet optique (OPD) qui se produit pendant un changement de relation de position du système étant corrigée par une fibre supplémentaire variable ou une ligne de retard.

5. Système d'examen ophtalmologique (100) qui comprend un dispositif de tomographie par cohérence optique (OCT), et contient

- un dispositif destiné à générer un faisceau d'examen (405),
- un dispositif d'analyse pour l'analyse du faisceau d'examen (406) renvoyé par une structure d'une cible et destiné à générer des données d'image d'une image de la structure de la cible
- un dispositif de guidage de faisceau destiné à guider le faisceau d'examen (406) depuis le dispositif de génération (405) du faisceau d'examen vers la cible et depuis la cible vers le dispositif d'analyse (402), une longueur de trajet optique du faisceau d'examen (406) dans le dispositif de guidage de faisceau étant variable de manière discrète ou continue,
- un dispositif de commande (500) destiné à commander le système d'examen ophtalmologique,
- la variation discrète ou continue de la longueur de trajet optique en fonction du temps étant connue ou pouvant être mesurée au moyen d'un arrangement de référence, et
- le dispositif de commande possédant une unité de correction, conçue pour corriger directement l'image ou les données d'image de l'image en fonction de la variation de la longueur de trajet optique (OPD) du faisceau d'examen (406) (406) dans le dispositif de guidage de faisceau,

l'unité de correction étant conçue pour corriger directement un cisaillement de l'image de tomographie par cohérence optique qui se produit en fonction de la variation de la longueur de trajet optique (OPD),
et dont le dispositif de tomographie par cohérence optique comprend notamment un dispositif d'OCT « en domaine temporel » (TD) ou d'OCT à « source balayée » (SS), et l'unité de correction est conçue pour corriger directement un décalage de l'image de tomographie par cohérence optique en fonction de la vitesse de variation de la longueur de trajet optique (dOPD/dt).

6. Système d'examen ophtalmologique (100) selon la revendication 5, comprenant en outre un élément de correction ajustable destiné à compenser la variation de la longueur de trajet optique (OPD).

7. Système d'examen ophtalmologique (100) selon la revendication 5 ou 6,

- une polarisation du faisceau d'examen (406) dans le dispositif de guidage de faisceau étant variable de manière discrète ou continue,
- la variation discrète ou continue de la polarisation en fonction du temps étant connue ou pouvant être mesurée au moyen d'un arrangement de référence, et
- le système d'examen ophtalmologique (100) possédant un élément de correction ajustable destiné à compenser la variation de la polarisation et/ou le dispositif de commande possédant une unité de correction conçue pour corriger directement l'image ou les données d'image de l'image en fonction de la variation de la polarisation du faisceau d'examen (406) dans le dispositif de guidage de faisceau.

8. Système d'examen ophtalmologique (100) selon la revendication 6, l'élément de correction ajustable comprenant une ligne à retard destinée à compenser la variation de la longueur de trajet optique (OPD) du faisceau d'examen (406) et l'élément de correction ajustable étant inclus dans le dispositif de guidage de faisceau et/ou dans un dispositif de guidage de faisceau alternatif pour un faisceau de référence.

9. Système d'examen ophtalmologique (100) selon la revendication 7, l'élément de correction ajustable comprenant deux palettes mobiles (460) ou une plaque à retard de phase pour compenser la variation de polarisation, et l'élément de correction ajustable étant inclus dans le dispositif de guidage de faisceau et/ou dans un dispositif de guidage de faisceau alternatif pour un faisceau de référence.

10. Système d'examen ophtalmologique (100) selon l'une des revendications 5 à 7, lequel contient en outre un dispositif d'affichage destiné à visualiser l'image à l'aide des données d'image de l'image d'une structure (950) de la cible (900).

11. Système d'examen ophtalmologique (100) selon l'une des revendications 5 à 10, le dispositif de guidage de faisceau possédant un système dont la relation de position interne de ses composants peut être modifiée et une fibre optique (410).

12. Système d'examen ophtalmologique (100) selon l'une des revendications 5 à 11, le dispositif de guidage du faisceau possédant un système dont la relation de position interne de ses composants peut être modifiée et au moins une partie du dispositif de guidage du faisceau étant conçue pour guider librement le faisceau d'examen à travers le système dont la relation de position interne de ses composants peut être modifiée.

13. Système d'examen ophtalmologique (100) selon l'une des revendications 5 à 12, dont le dispositif de guidage de faisceau comprend une optique mobile, notamment une lentille d'objectif (225) mobile latéralement.

14. Système d'examen ophtalmologique (100) selon l'une des revendications 5 à 13, dont le dispositif pour la tomographie par cohérence optique (OCT) est divisé en systèmes partiels fermés, interchangeables indépendamment les uns des autres et pouvant être corrigés individuellement avec des transitions définies, de préférence en un premier système partiel qui contient un interféromètre (402) et un deuxième système partiel, qui comprend une fibre optique (410) pourvue d'un collimateur (450) ou, en alternative, de préférence en un premier système partiel qui contient un interféromètre (402), un deuxième système partiel qui contient une fibre optique (410) longue qui passe à travers un système dont la relation de position interne de ses composants peut être modifiée, et en un troisième système partiel qui comprend une fibre optique (410) courte et un collimateur (450).

15. Système d'examen ophtalmologique (100) selon l'une des revendications 5 à 14, qui comprend un dispositif laser de thérapie (200), un dispositif de génération de faisceau d'examen (406) étant également conçu pour générer le faisceau laser de thérapie ou un dispositif indépendant de génération (210) du faisceau laser de thérapie étant inclus en plus du dispositif de génération (405) du faisceau d'examen.

**Fig. 1**

Fig. 2

X (Max)

**G**$_X$

240
(X→Y)

224
(Y→Z)

430

225

900

406

**Fig. 3a**

X (Min)

**G**$_X$

240
(X→Y)

224
(Y→Z)

430

225

900

406

**Fig. 3b**

Y (Max)

240
(X→Y)

224 (Y→Z)

**G**$_Y$

430

225

900

406

**Fig. 3c**

Y (Min)

**G**$_Y$

240
(X→Y)

224 (Y→Z)

430

225

900

406

**Fig. 3d**

EP 3 644 888 B1

27

X

Y

240

224

406

OPD = X + Y

**Fig. 4**

900

950

900'

950'

900"-1

950"-1

950'-2

900"-2

**Fig. 5a**　　　　**Fig. 5b**　　　　**Fig. 5c**

900

950

**Fig. 6a**

900

950"

950'

**Fig. 6b**

detector signal:
I=sin(k·z)

K = 2π/λ, k-sweep

z, OPD

z=const.= z1 → f=2
reco: z=2

z=const.=z2 → f=5
reco: z=5

z: z1..z2 ~ k → f = 14
**reco: z=14**

z: z1..z2; k=const. → f= 9    z: z1..z2; k=const. → f= 12

## Fig. 7

Fig. 8

**Fig. 9**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2016058931 A2
- DE 102014115153 A1
- WO 2010098204 A1
- DE 102010019657 A1
- DE 102004028204 B3